# EUROPEAN PATENT APPLICATION

(11) **EP 4 733 297 A1**
(43) Date of publication of application: **29.04.2026**
(21) Application number: 24826021.8
(22) Date of filing: 21.06.2024
(51) Int. Cl.: C07D 239/14, A61K 31/505, A61K 45/00, A61P 1/16, A61P 11/00, A61P 13/12, A61P 43/00

(54) **INTEGRIN-INHIBITING SMALL MOLECULE COMPOUND**

(30) Priority: 22.06.2023 JP 2023102623
(71) Applicant: Antibody Therapeutics, Inc., Hiroshima-shi, Hiroshima 731-5135 (JP)
(72) Inventor: YOKOSAKI, Yasuyuki, Hiroshima-shi, Hiroshima 731-5135 (JP); NISHIMICHI, Norihisa, Hiroshima-shi, Hiroshima 731-5135 (JP)
(74) Representative: Plasseraud IP
(86) International application number: PCT/JP2024/022642
(87) International publication number: WO 2024/262620

(57) **Abstract**

An object of the present invention is to provide a novel group of compounds having an activity of inhibiting integrin function, pharmaceutical compositions, and therapeutic agents or prophylactic agents for a disease accompanied by fibrosis. The present invention provides compounds represented by the following formula (I) or a salt thereof, wherein A is C-OH or N, B is C-H, C-COOH, C-CH₃, C-Br, or N, X is -SFs or -Si(CH₃)₃, Y is -F or -OH, and * indicates an asymmetric carbon atom, and further provides integrin inhibitors, pharmaceutical compositions and therapeutic agents or prophylactic agents for a disease accompanied by fibrosis, using them.

## Description

### [TECHNICAL FIELD]

The present invention relates to low-molecular-weight organic compounds or salts thereof having an integrin inhibitory function, and further relates to integrin inhibitors, pharmaceutical compositions, and therapeutic agents or prophylactic agents for diseases accompanied by fibrosis, using the compounds.

### [BACKGROUND ART]

Fibrosis of organs and tissues, which is also a physiological phenomenon accompanying aging, often shows pathological spread. Starting from the fact that there is no therapeutic drug, it stands as a major unresolved problem in medical care. Pathological fibrosis is well known on an organ-by-organ basis. The lung and liver are particularly representative and their fibrosis have their own disease names, namely, interstitial pneumonia and liver cirrhosis, respectively. It is chronically, but occasionally acutely, progressive. The fibrosis occurs widespread, and does not return to its original state. The pathological findings of fibrosis are excessive deposition of extracellular matrix proteins such as collagen, which replaces organspecific parenchymal cells, and eventually the organ loses its function. Usually, this progressive process gives patients not only pain but also psychological fear. Furthermore, even minor fibrosis, when accumulated, impairs organs and becomes a disease factor for the heart (heart failure from fibrosis of myocardium) and the kidney (proteinuria from fibrosis of interstitium). For these reasons, there is a large need for a therapeutic drug that stops the progression of fibrosis and returns it to its original state, and a large contribution is expected not only to cure specific fibroses but also to extension of healthy life expectancy and the like. Global development competition is heating up, but there is still no therapeutic drug. According to an estimate from epidemiological investigations, for the liver alone, it is expected that at least about 5 million people will develop the disease in Japan over the next 10 years.

There are various matrix proteins, besides collagen, which are primary constituent molecules of the fibrotic tissue; however, they are produced from activated and/or differentiated fibroblasts, as instructed by TGFβ signaling. From such a pathological condition, involvement of TGFβ and the 24 types of integrin family that are matrix receptors is strongly suspected. TGFβ is scattered and stands by as a latent type in accumulated extracellular matrices, becomes an active type as necessary, and gives surrounding cells essential instructions for formation of the pathological condition of fibrosis (secretion of matrix, activation of fibroblasts and differentiation into myofibroblasts, and the like). However, a therapeutic strategy of directly inhibiting the highly functional molecule TGFβ as a target is difficult to develop from aspects such as side effects. On the other hand, integrin is a protein present in the plasma membrane on the cell surface, and is capable of connecting the cytoskeleton present inside the cell and the extracellular matrix present outside the cell. Its structure is a heterodimer in which two subunits, an α chain and a β chain, associate at 1:1; at least 18 types of α chains and at least 8 types of β chains have been reported; and it is expressed, for example, as α1β1 (i.e., an integrin composed of an α chain called α1 and a β chain called β1) using the types of the constituting α chain and β chain. Integrin is known to mediate the transformation of TGFβ from a latent type to an active type. This "integrin activation" occurs by binding of integrin to latent-type TGFβ and, thereby, removing the sheath (LAP; latency associated protein) worn by active-type TGFβ by tension. LAP is an N-terminal portion of pro-TGFβ coded by the same gene as TGFβ. The binding site is the Arg-Gly-Asp (RGD) sequence possessed by LAP, and integrins that bind to this sequence (RGD-recognizing integrins) are only 8 types out of all 24 types. However, although there are integrins that cannot activate TGFβ even if they bind thereto, αv-integrins (αvβ1, αvβ3, αvβ5, αvβ6, αvβ8), which account for five of the 24 types, all recognize RGD and function in TGFβ activation, and thus their inhibitors attract a high level of attention as antifibrotic drugs. Among them, Pliant Therapeutics' αvβ1/αvβ6 dual inhibitor PLN-74809 was reported in July 2022 to have proven efficacy in early Phase 2a of a clinical trial targeting interstitial pneumonia, and is attracting great attention (Non-Patent Literature 1).

The first step of TGFβ activation by integrin is binding to LAP. LAP has an RGD sequence to which αv-integrins exclusively preferentially bind. TGFβ becomes free by the binding as a trigger,. Besides αv-integrins, three types of RGD-recognizing integrins are known; among them, existence of TGFβ activation activity has been confirmed for α8β1, and it also has an action of differentiating fibroblasts into myofibroblasts. On the other hand, the expression sites of α8β1 are overwhelmingly limited compared with other integrins, being almost limited to fibroblasts, and the expression level is enhanced by fibrotic stimulation. In this manner, not only its function but also its expression strongly associates strongly with the progression of fibrosis. These facts suggest that the function of α8β1 plays a role in fibrosis formation. In fact, an α8β1 inhibitory antibody has been shown in the liver to suppress fibrosis (Non-Patent Literature 2).

### [PRIOR-ART REFERENCES]

### [NON-PATENT LITERATURE]

[Non-Patent Literature 1] Pliant Therapeutics Announces Positive Safety and Efficacy Data from Phase 2a INTEGRIS-IPF Clinical Trial of PLN-74809 in Patients with Idiopathic Pulmonary Fibrosis, [online], July 10, 2022, Pliant Therapeutics press release, Internet URL:'https://ir.pliantrx.com/node/7886/pdf'
[Non-Patent Literature 2] Norihisa Nishimichi and seven others, Journal of Pathology, January 12, 2021, Vol. 253, issue 4, pp. 366-373

### [SUMMARY OF THE INVENTION]

### [PROBLEMS TO BE SOLVED BY THE INVENTION]

In view of the above conventional situation, an object of the present invention is to provide a novel group of compounds having an activity of inhibiting integrin function, a pharmaceutical composition, and a therapeutic agent or prophylactic agent for a disease accompanied by fibrosis.

### [MEANS FOR SOLVING THE PROBLEMS]

The present invention provides a compound represented by the following formula (I) or a salt thereof.

A compound represented by formula (I) or a salt thereof: 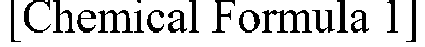 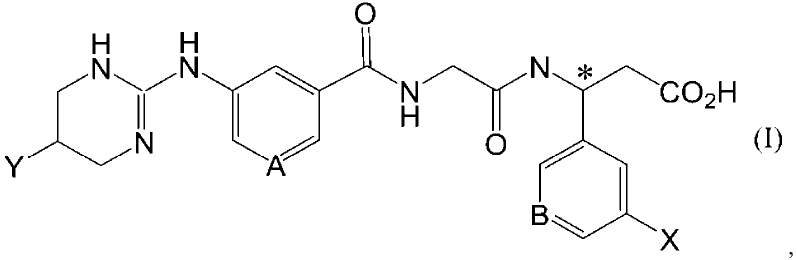
wherein A is C-OH or N,
B is C-H, C-COOH, C-CH₃, C-Br, or N,
X is -SF₅ or -Si(CH₃)₃,
Y is -F or -OH, and
* indicates an asymmetric carbon atom.

In the compound or a salt thereof according to the present invention, it is preferable that X is -SF₅ and B is C-H, C-CH₃, or C-Br. In this case, Y is preferably -F, and in addition, A is preferably C-OH.

In the compound or a salt thereof according to the present invention, X may be - Si(CH₃)₃, and B may be C-COOH or N.

Further, the present invention provides an integrin inhibitor containing any of the above compounds or a pharmaceutically acceptable salt thereof.

Further, the present invention provides a pharmaceutical composition containing any of the above compounds or a pharmaceutically acceptable salt thereof, and a pharmaceutically acceptable carrier.

Further, the present invention provides a therapeutic agent or prophylactic agent for a disease accompanied by fibrosis, containing, as an active ingredient, any of the above compounds or a pharmaceutically acceptable salt thereof. In this case, a disease accompanied by fibrosis may be kidney fibrosis, lung fibrosis (for example, interstitial pneumonia), or liver fibrosis (for example, liver cirrhosis), and the therapeutic agent or prophylactic agent for a disease accompanied by fibrosis may be used in combination with at least one drug selected from drugs classified as therapeutic agents or prophylactic agents for diseases accompanied by fibrosis.

Further, the present invention provides a therapeutic agent or prophylactic agent for a disease accompanied by fibrosis comprising a combination of any of the above compounds or a pharmaceutically acceptable salt thereof and an active ingredient of at least one drug selected from drugs classified as therapeutic agents or prophylactic agents for diseases accompanied by fibrosis.

Further, the present invention provides any of the above compounds or a pharmaceutically acceptable salt thereof for use as a therapeutic drug or prophylactic drug for a disease accompanied by fibrosis.

Further, the present invention provides use of any of the above compounds or a pharmaceutically acceptable salt thereof for the manufacture of a medicament for treating or preventing a disease accompanied by fibrosis.
Further, the present invention provides a method for treating or preventing a disease accompanied by fibrosis by administering any of the above compounds or a pharmaceutically acceptable salt thereof to a patient.

The present invention includes the following embodiment.

[1] A compound represented by formula (I) or a salt thereof: 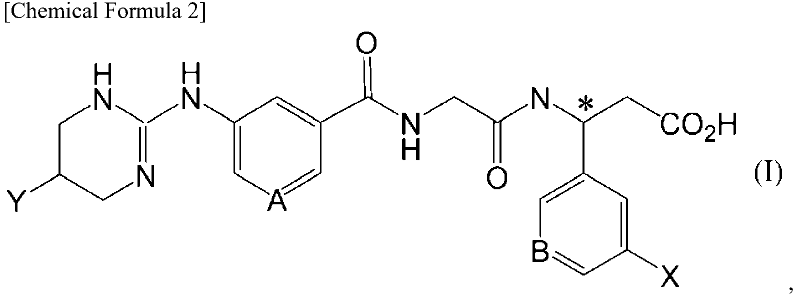
   wherein A is C-OH or N,
   B is C-H, C-COOH, C-CH₃, C-Br, or N,
   X is -SF₅ or -Si(CH₃)₃,
   Y is -F or -OH, and
   * indicates an asymmetric carbon atom.
[2] The compound or a salt thereof according to [1], wherein, in formula (I), X is -SF₅ and B is C-H, C-CH₃, or C-Br.
[3] The compound or a salt thereof according to [2], wherein, in formula (I), Y is -F.
[4] The compound or a salt thereof according to [3], wherein, in formula (I), A is C-OH.
[5] The compound or a salt thereof according to [1], wherein, in formula (I), X is -Si(CH₃)₃ and B is C-COOH or N.
[6] An integrin inhibitor comprising the compound or a pharmaceutically acceptable salt thereof according to any one of [1] to [5].
[7] A pharmaceutical composition comprising the compound or a pharmaceutically acceptable salt thereof according to any one of [1] to [5], and a pharmaceutically acceptable carrier.
[8] A therapeutic agent or prophylactic agent for a disease accompanied by fibrosis, containing, as an active ingredient, the compound or a pharmaceutically acceptable salt thereof according to any one of [1] to [5].
[9] The therapeutic agent or prophylactic agent for a disease accompanied by fibrosis according to [8], wherein the disease accompanied by fibrosis is kidney fibrosis, lung fibrosis, or liver fibrosis.
[10] The therapeutic agent or prophylactic agent for a disease accompanied by fibrosis according to [8], for treating or preventing a disease accompanied by fibrosis in combination with at least one drug selected from drugs classified as therapeutic agents or prophylactic agents for diseases accompanied by fibrosis.
[11] A therapeutic agent or prophylactic agent for a disease accompanied by fibrosis comprising a combination of the compound or a pharmaceutically acceptable salt thereof according to any one of [1] to [5], and an active ingredient of at least one drug selected from drugs classified as therapeutic agents or prophylactic agents for diseases accompanied by fibrosis.
[12] The compound or a pharmaceutically acceptable salt thereof according to any one of [1] to [5], for use as a therapeutic drug or prophylactic drug for a disease accompanied by fibrosis.
[13] Use of the compound or a pharmaceutically acceptable salt thereof according to any one of [1] to [5], for the manufacture of a medicament for treating or preventing a disease accompanied by fibrosis.
[14] A method for treating or preventing a disease accompanied by fibrosis by administering the compound or a pharmaceutically acceptable salt thereof according to any one of [1] to [5] to a patient.

### [EFFECTS OF THE INVENTION]

The compound or a salt thereof and the integrin inhibitor according to the present invention have an activity of inhibiting integrin function, and thus can suppress transformation, mediated by integrin, of TGFβ from a latent type to an active type. Further, they can be expected as a therapeutic agent, a prophylactic agent, or a prodrug thereof for a disease accompanied by fibrosis in which active-type TGFβ is involved, and can be used for development of pharmaceuticals for use in a disease accompanied by fibrosis.

The pharmaceutical composition of the present invention exerts an effect of treating or preventing a disease accompanied by fibrosis, with inhibition of integrin function as one mechanism of action.

The therapeutic agent or prophylactic agent for a disease accompanied by fibrosis of the present invention exerts an effect of alleviating symptoms of diseases accompanied by fibrosis, such as kidney fibrosis, interstitial pneumonia, or liver cirrhosis, or preventing onset of a disease accompanied by fibrosis.

### [BRIEF DESCRIPTION OF THE DRAWINGS]

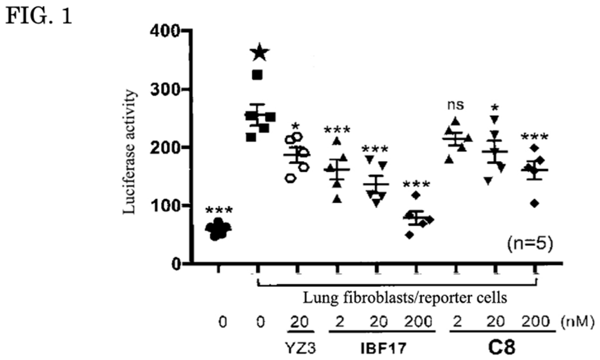

[Fig. 1] Fig. 1 shows the effect on TGFβ activation on the cell surface of lung fibroblasts. From the left, measurement results of luciferase activity in the case of reporter cells alone, mixed culture of reporter cells and lung fibroblasts, and addition of IBF17 and C8 are shown. The symbol * indicates the control. *, **, and *** indicate P<0.05, P<0.01, and P<0.001, respectively, and n.s. indicates no significant difference (unless otherwise specified, the same applies in the following drawings). In addition, significant differences in this experiment were tested by Dunnett's multiple comparisons test against the control.
[Fig. 2] Fig. 2 shows the effect on differentiation of fibroblasts into myofibroblasts. Results of western blotting using α-SMA and GAPDH when IBF-17 and C8 (Comparative Example) were added are shown.
[Fig. 3] Fig. 3 shows the effect on differentiation of fibroblasts into myofibroblasts. Relative values of band intensity of α-SMA when IBF-17 and C8 (Comparative Example) were added are shown. Significant differences were tested by Dunnett's multiple comparisons test against TGFβ addition only (compound 0 nM).
[Fig. 4] Fig. 4 shows a dosing schedule of an 8-week administration experiment in a carbon tetrachloride (CCl₄) administration model.
[Fig. 5] Fig. 5 shows hydroxyproline quantification results of liver tissue in the same experiment. Significant differences were tested by Student's t-test against CCl₄ alone (0 mg/kg).
[Fig. 6] Fig. 6 shows comparison of Sirius red staining area of tissue and photographs after staining in the same experiment. Significant differences were tested by Bonferroni's Multiple Comparison test against CCl4 alone.
[Fig. 7] Fig. 7 shows mRNA expression levels (relative intensity) of fibrosis marker genes (Col1a1 gene and Acta2 gene) in the same experiment.
[Fig. 8] Fig. 8 shows a dosing schedule of a 3-day administration experiment in a carbon tetrachloride (CCl₄) administration model.
[Fig. 9] Fig. 9 shows mRNA expression levels (relative intensity) in qPCR in the same experiment. Significant differences were tested by Dunnett's multiple comparisons test against CCl4 (Veh).
[Fig. 10] Fig. 10 shows a dosing schedule of an 8-week administration experiment in a NASH model.
[Fig. 11] Fig. 11 shows hydroxyproline quantification results of liver tissue in the same experiment. Significant differences were tested by Student's t-test against CDAHFD-feeding alone (0 mg/kg).
[Fig. 12] (A) and (B) show changes in body weight of mice and liver weight after 56 weeks in the same experiment.
[Fig. 13] Fig. 13 shows comparison of Sirius red staining area of tissue, and photographs after staining in the same experiment. Significant differences were tested by Bonferroni's Multiple Comparison test against CDAHFD-feeding alone (0 mg/kg).
[Fig. 14] Fig. 14 shows mRNA expression levels (relative intensity) of genes regarded as fibrosis markers (Acta2 gene, Col1a1 gene, and Timp-1 gene) in the same experiment.
[Fig. 15] Fig. 15 shows a dosing schedule of a 10-day administration experiment in a unilateral ureteral obstruction (UUO) model.
[Fig. 16] Fig. 16 shows mRNA expression levels (relative intensity) in qPCR in the same experiment. Significant differences were tested by Dunnett's multiple comparisons test against UUO (Veh).
[Fig. 17] Fig. 17 shows the area under the blood concentration-time curve and margin when the compound of the present invention was administered.
[Fig. 18] Fig. 18 shows a dosing schedule of a 15-day administration experiment in a carbon tetrachloride (CCl₄) administration model.
[Fig. 19] Fig. 19 shows hydroxyproline quantification results of liver tissue in the same experiment. Significant differences were tested by Dunnett's multiple comparisons test against CCl₄ (Vehicle).
[Fig. 20] Fig. 20 shows a dosing schedule of a 10-day administration experiment in a bleomycin administration model.
[Fig. 21] Fig. 21 shows mRNA expression levels (relative intensity) of fibrosis-related genes (Acta2, Col1a1, Col3a1, Tnc) in the same experiment. Significant differences were tested by Dunnett's multiple comparisons test against Bleo (vehicle).
[Fig. 22] Fig. 22 shows comparison of Masson-trichrome staining area of lung tissue and photographs after staining in the same experiment. Significant differences were tested by Dunnett's multiple comparisons test against Bleo (vehicle).

### [EMBODIMENTS FOR CARRYING OUT THE INVENTION]

Hereinafter, the present invention will be described, using exemplary embodiments as examples, together with preferred methods and materials that can be used in practicing the present invention; however, the present invention is not limited to the aspects described below. Unless otherwise specified in the text, all technical and scientific terms used in this specification have the same meaning as commonly understood by those skilled in the art to which the present invention pertains. Further, any materials and methods equivalent or similar to those described in this specification can likewise be used in practicing the present invention. Further, all publications and patents cited herein in connection with the present invention are cited herein and constitute part of this specification, as indicating, for example, methods, materials, and the like that can be used in the present invention.

In this specification, the expression "A - B" indicating a numerical range means a numerical range including endpoints A and B. The same applies to "A through B." Further, in this specification, "about" is used to mean allowing ± 10%.

### 1. Compound or Salt Thereof

### 1-1. Structure of Compound

The compound of the present invention has a chemical structure represented by the following formula (I). 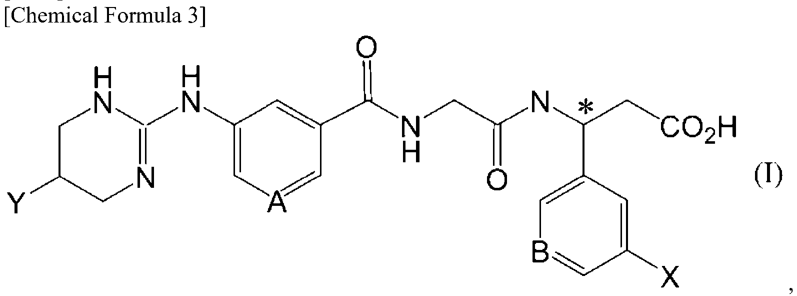
wherein A is C-OH or N,
B is C-H, C-COOH, C-CH₃, C-Br, or N,
X is -SF₅ or -Si(CH₃)₃, and
Y is -F or -OH.)

In formula (I), since an asymmetric carbon atom is present at the position indicated by *, the compound of the present invention has two optical isomers represented by the following formula (I-I) and formula (I-II), and may be the S form only, the R form only, or a racemate. In the following description, a structural formula of the S form is described; however, the present invention is not limited thereto.

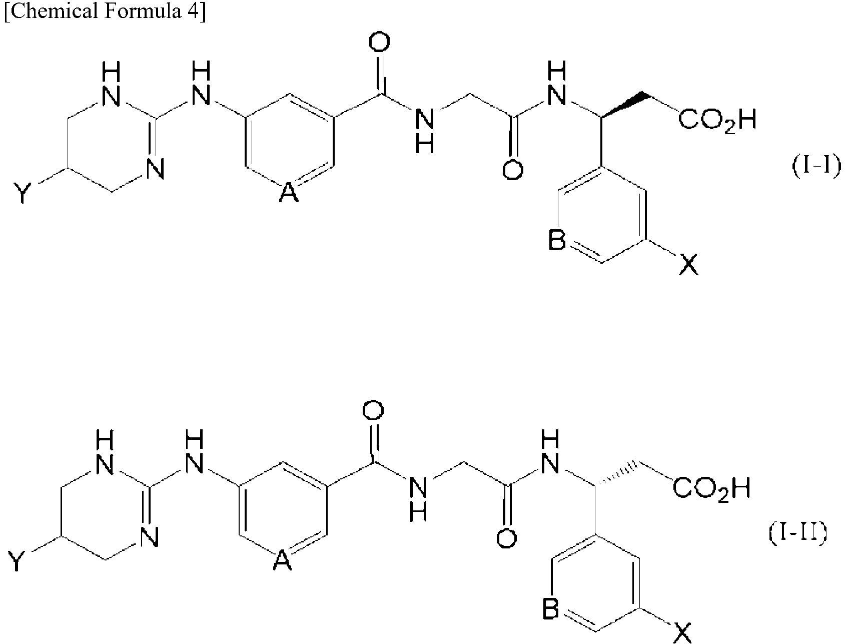

In formula (I-I), when A is C-OH, the chemical structure is represented by the following formula (II), and when A is N, the chemical structure is represented by the following formula (III).

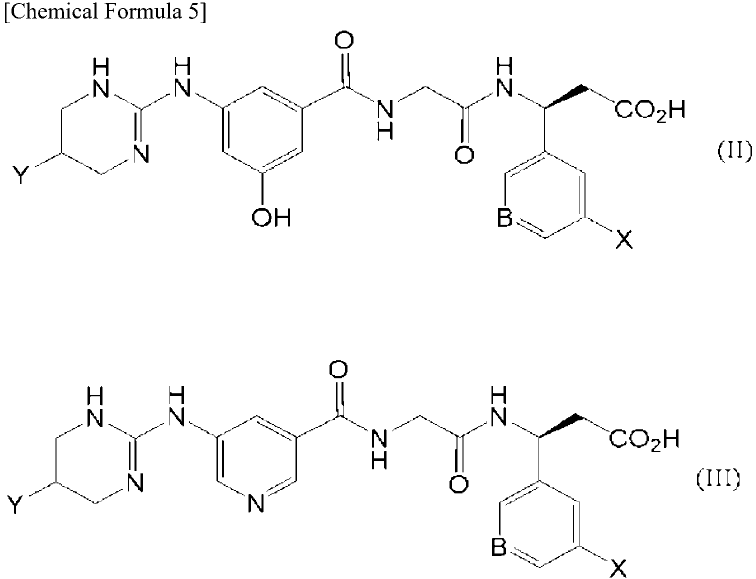

In formula (I-I), when B is C-H, the chemical structure is represented by the following formula (IV); when B is C-COOH, the chemical structure is represented by the following formula (V); when B is C-CH₃, the chemical structure is represented by the following formula (VI); when B is C-Br, the chemical structure is represented by the following formula (VII); and when B is N, the chemical structure is represented by the following formula (VIII).

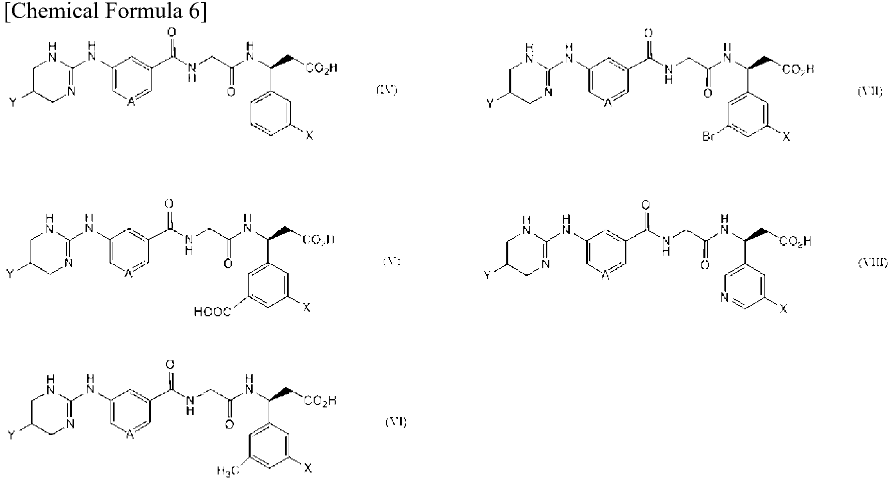

In formula (I-I), when X is -SF₅, the chemical structure is represented by the following formula (IX), and when X is -Si(CH₃)₃, the chemical structure is represented by the following formula (X).

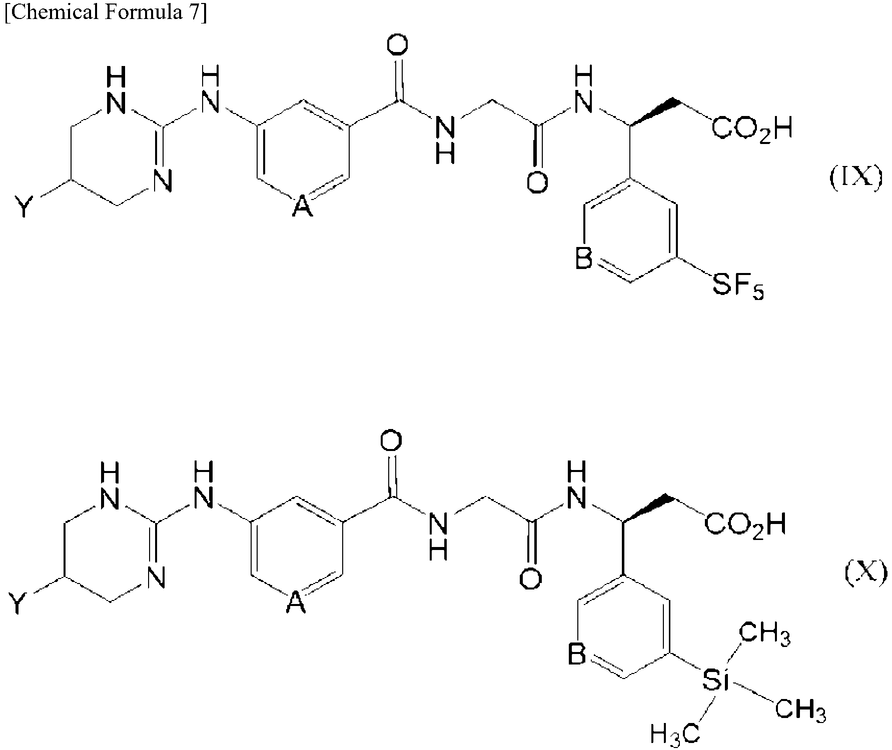

In formula (I-I), when Y is -F, the chemical structure is represented by the following formula (XI), and when Y is -OH, the chemical structure is represented by the following formula (XII).

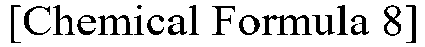

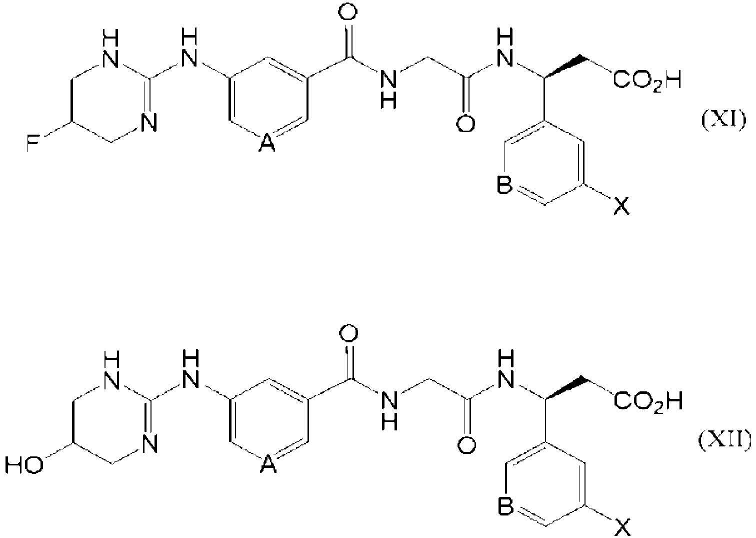

### 1-2. Specific Examples of Compounds

Table 1 shows specific examples of compounds having the chemical structure represented by the above formula (I).

**[Table 1]**

| Specific examples of compounds | |
|---|---|
| Compound No. | Structural formula |
| IBF-17 | 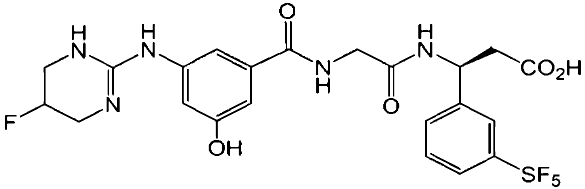 |
| IBF-21 | 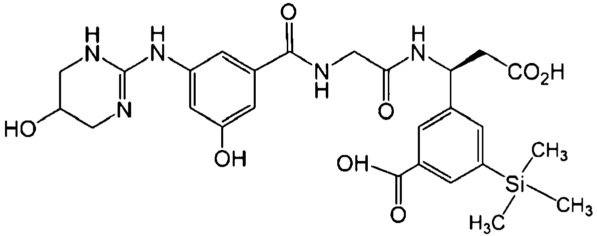 |
| IBF-18 | 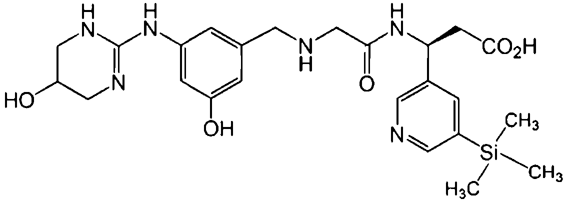 |
| IBF-20 | 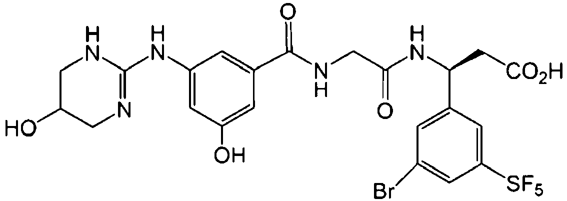 |
| IBF-13 | 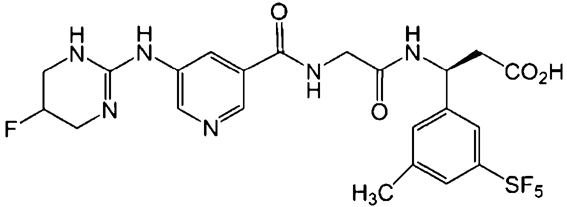 |

In IBF-17, A is C-OH, B is C-H, X is -SF₅, and Y is -F.

In IBF-21, A is C-OH, B is C-COOH, X is -Si(CH₃)₃, and Y is -OH.

In IBF-18, A is C-OH, B is N, X is -Si(CH₃)₃, and Y is -OH.

In IBF-20, A is C-OH, B is C-Br, X is -SF₅, and Y is -OH.

In IBF-13, A is N, B is C-CH₃, X is -SF₅, and Y is -F.

### 1-3. Salts of Compound

Salts of the compound of the present invention may include, for example, salts with inorganic bases, salts with organic bases, salts with inorganic acids, salts with organic acids, salts with acidic amino acids or basic amino acids, and the like. When the compound of the present invention represented by formula (I) has an acidic functional group, it can be a salt with an inorganic base, an organic base, or a basic amino acid. Further, when the compound of the present invention represented by formula (I) has a basic functional group, it can be a salt with an inorganic acid, an organic acid, or an acidic amino acid.

Examples of salts with inorganic bases include, without limitation, for example, sodium salts, potassium salts, ammonium salts, calcium salts, magnesium salts, barium salts, aluminum salts, and the like. Examples of salts with organic bases include, without limitation, for example, salts with trimethylamine, triethylamine, pyridine, picoline, 2,6-lutidine, ethanolamine, diethanolamine, triethanolamine, tromethamine [tris(hydroxymethyl)aminomethane], tert-butylamine, cyclohexylamine, dicyclohexylamine, N,N-dibenzylethylamine, and the like. Examples of salts with inorganic acids include, without limitation, for example, salts with hydrochloric acid, phosphoric acid, sulfuric acid, hydrobromic acid, hydroiodic acid, nitric acid, and the like. Examples of salts with organic acids include, without limitation, for example, salts with acetic acid, phthalic acid, fumaric acid, oxalic acid, trifluoroacetic acid, citric acid, maleic acid, succinic acid, malic acid, acetic acid, formic acid, propionic acid, benzoic acid, methanesulfonic acid, benzenesulfonic acid, para-toluenesulfonic acid, camphorsulfonic acid, and the like. Examples of salts with acidic amino acids include, without limitation, for example, salts with aspartic acid and glutamic acid, and examples of salts with basic amino acids include, for example, salts with arginine, lysine, ornithine, and the like.

Starting materials and intermediates of the compound of the present invention may form salts. Salts of starting materials and intermediates are preferably conventional nontoxic salts. Examples thereof include salts with bases such as inorganic base salts (for example, sodium salts, potassium salts, ammonium salts, calcium salts, magnesium salts, barium salts, aluminum salts, and the like) and organic base salts (for example, trimethylamine salts, triethylamine salts, pyridine salts, picoline salts, dicyclohexylamine salts, N,N-dibenzylethylamine salts); salts with acids such as inorganic acid salts (for example, hydrochlorides, hydrobromides, hydroiodides, sulfates, nitrates, phosphates, and the like) and organic acid salts (for example, acetates, trifluoroacetates, maleates, fumarates, citrates, tartrates, methanesulfonates, benzenesulfonates, formates, para-toluenesulfonates, and the like); salts with amino acids (for example, arginine, aspartic acid, glutamic acid, and the like); and the like, and those skilled in the art can appropriately select them.

### 1-4. Method for Synthesizing Compound

The above compounds can be synthesized, without limitation, for example, by synthesizing, in the chemical structure represented by formula (I), the aromatic moiety on the right side and the two cyclic structure moieties (cyclic guanidine and aromatic) on the left side, and coupling them. General synthetic procedures thereof are described below in Schemes I to III; however, the following schemes and examples are merely examples of the present invention and are not intended to be limited to such configurations. Those skilled in the art can synthesize the compound of the present invention by appropriately modifying the schemes based on the disclosure of this specification, existing commercially available chemicals, and known synthesis examples.

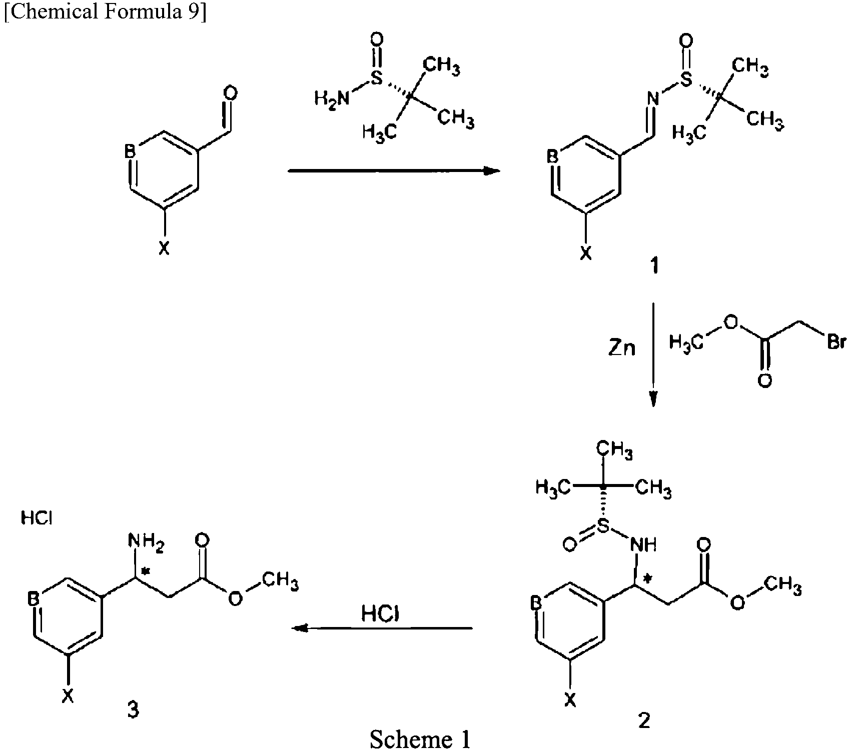

Scheme I is an example of a procedure for synthesizing the aromatic moiety on the right side of the chemical structure represented by formula (I). Intermediate 1 was synthesized by carrying out a condensation reaction of 2-methylpropane-2-sulfinamide with an appropriate aromatic compound containing an aldehyde group. Next, methyl bromoacetate was nucleophilically added to Intermediate 1 in the presence of zinc to synthesize Intermediate 2. By treating Intermediate 2 with hydrochloric acid, the sulfinyl group was removed to synthesize the hydrochloride of Intermediate 3.

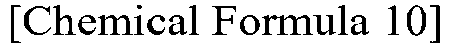

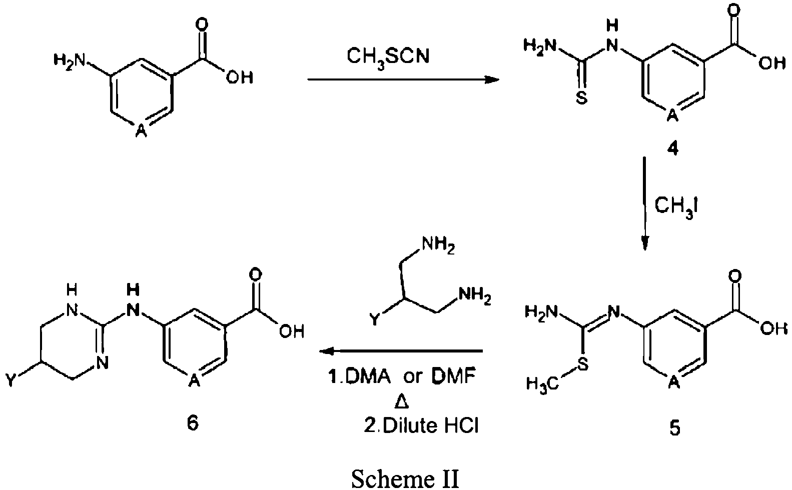

Scheme II is an example of a procedure for synthesizing the two cyclic structure moieties on the left side of the chemical structure represented by formula (I). Intermediate 4 was synthesized by reacting methyl isothiocyanate with an appropriate aromatic compound containing an amino group and a carboxylic acid group. Next, iodomethane is reacted with the thio group of Intermediate 4 to synthesize Intermediate 5. A suitable 1,3-diamino-2-propane derivative is reacted with Intermediate 5 in high-temperature N,N-dimethylacetamide (DMA) or N,N-dimethylformamide (DMF). The HCl salt may be obtained by lyophilization from dilute hydrochloric acid.

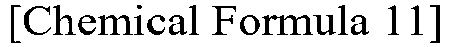

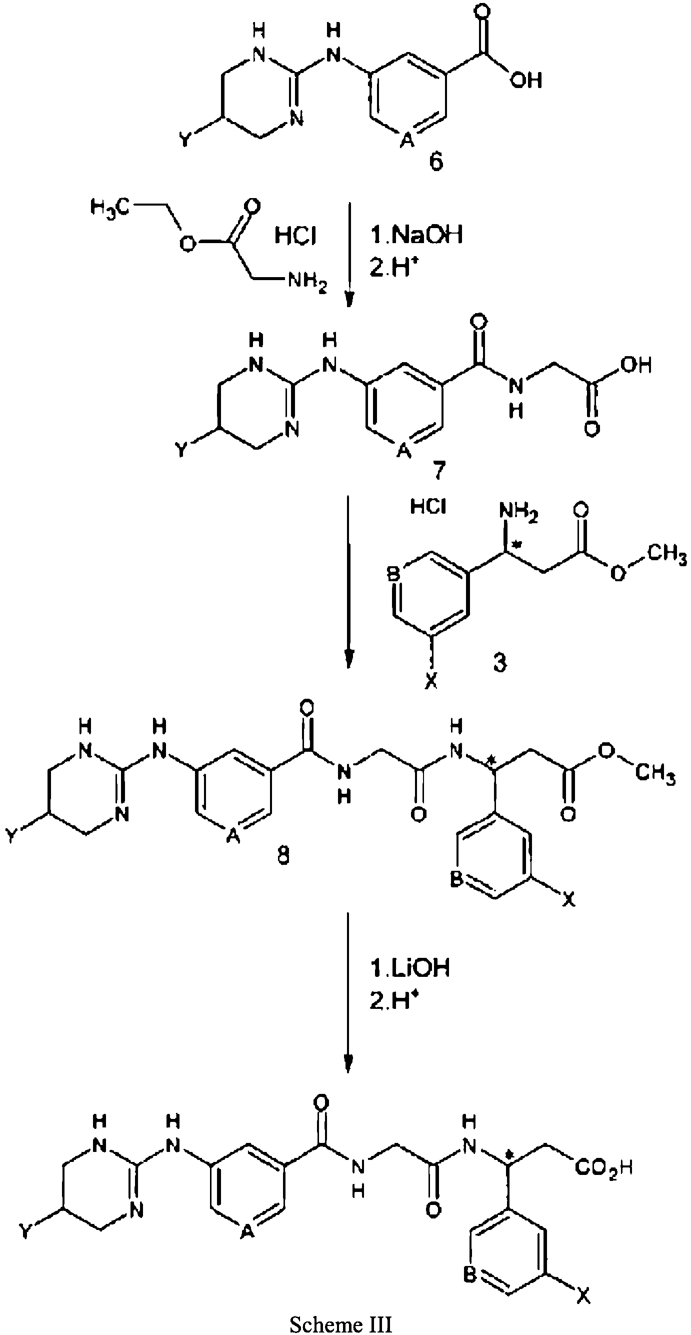

Scheme III is an example of a procedure for synthesizing the compound represented by formula (I) by coupling Intermediate 3 of Scheme I and Intermediate 6 of Scheme II. In Scheme III, Intermediate 6 is activated for coupling using a known method and reacted with ethyl glycinate hydrochloride, and, by treatment with a base such as NaOH in an appropriate solvent (water, dioxane/water, or acetonitrile/water), the ester is hydrolyzed to an acid, followed by acidification, to synthesize Intermediate 7. Next, Intermediate 7 is activated for coupling using a known method and reacted with Intermediate 3 of Scheme I to synthesize Intermediate 8. Finally, by treating Intermediate 8 with a base such as LiOH in an appropriate solvent (dioxane/water or acetonitrile/water), the ester is hydrolyzed to an acid, followed by acidification, to synthesize the compound represented by formula (I). In Scheme III, glycine was added to Intermediate 6 and coupled with Intermediate 3; however, Intermediate 3 may be treated with activated Boc-glycine and coupled with Intermediate 6.

In the case of the compound IBF-17, it can be synthesized from the following compounds using Schemes I to III. Here, 3-(pentafluorosulfanyl)benzaldehyde (CAS No.: 401892-80-6) and 3-amino-5-hydroxybenzoic acid (CAS No.: 14206-69-0) are commercially available and can be obtained. 2-fluoropropane-1,3-diamine can be obtained, for example, by the synthesis method described in Example 1.

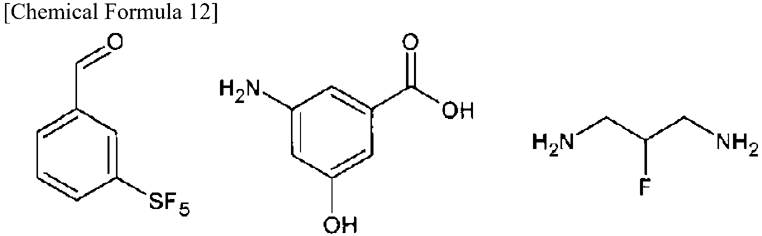

In the case of the compound IBF-21, it can be synthesized from the following compounds using Schemes I to III. Here, 3-amino-5-hydroxybenzoic acid and 1,3-diamino-2-propanol (CAS No.: 616-29-5) are commercially available and can be obtained. 3-carboxy-5-(trimethylsilyl)benzaldehyde can be synthesized by Scheme IV below.

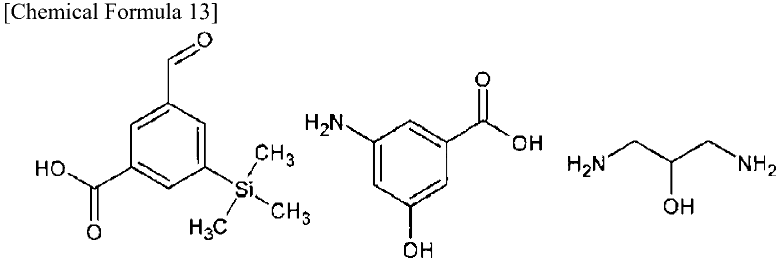

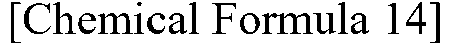

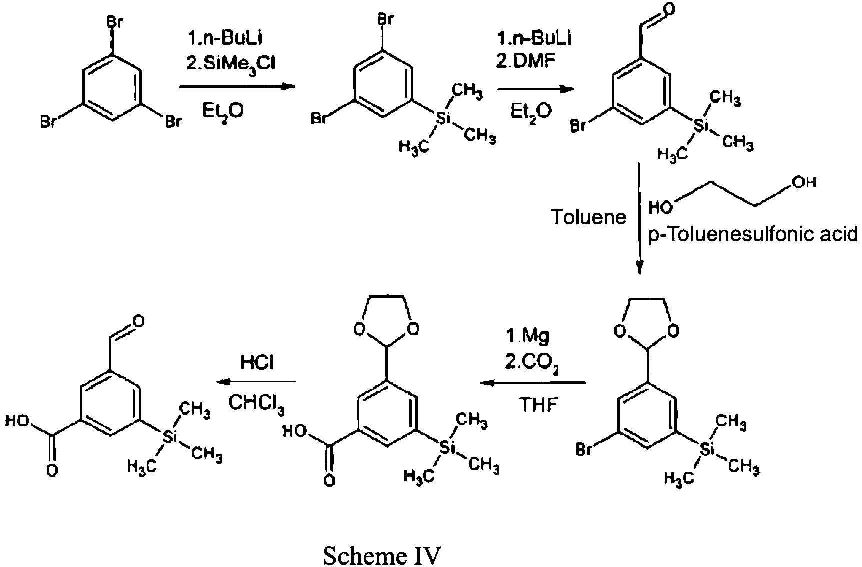

Scheme IV is an example of a procedure for synthesizing 3-carboxy-5-(trimethylsilyl)benzaldehyde from commercially available 1,3,5-tribromobenzene (CAS No.: 626-39-1) (see Hiroaki Horiuchi et al., "Silylation Improves the Photodynamic Activity of Tetraphenylporphyrin Derivatives In Vitro and In Vivo", CHEMISTRY A European Journal Supporting Information). First, n-butyllithium (n-BuLi) was added to 1,3,5-tribromobenzene in diethyl ether (Et₂O), and then chlorotrimethylsilane was added to synthesize 1,3-dibromo-5-(trimethylsilyl)benzene. Next, n-butyllithium (n-BuLi) was added to 1,3-dibromo-5-(trimethylsilyl)benzene in diethyl ether (Et₂O), and then N,N-dimethylformamide (DMF) was added to synthesize 3-bromo-5-(trimethylsilyl)benzaldehyde. Further, para-toluenesulfonic acid and ethylene glycol were added to 3-bromo-5-(trimethylsilyl)benzaldehyde in toluene to synthesize 2-(3-bromo-5-(trimethylsilyl)phenyl)-1,3-dioxolane. Then, 2-(3-bromo-5-(trimethylsilyl)phenyl)-1,3-dioxolane was added to activated Mg in dehydrated tetrahydrofuran (THF), and thereafter carbon dioxide gas was introduced to synthesize 2-(3-carboxy-5-(trimethylsilyl)phenyl)-1,3-dioxolane. Finally, 2-(3-carboxy-5-(trimethylsilyl)phenyl)-1,3-dioxolane was dissolved in chloroform (CHCl₃), HCl was added, and 3-carboxy-5-(trimethylsilyl)benzaldehyde was synthesized.

In the case of the compound IBF-18, it can be synthesized from the following compounds using Schemes I to III. Here, 3-pyridinecarboxaldehyde, 5-(trimethylsilyl) (CAS No.: 144056-15-5), 3-amino-5-hydroxybenzoic acid, and 1,3-diamino-2-propanol are all commercially available and can be obtained.

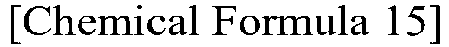

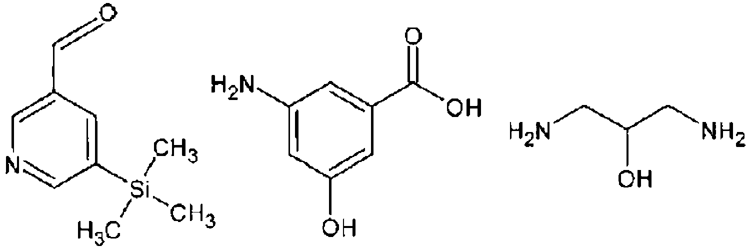

**In** the case of the compound IBF-20, it can be synthesized from the following compounds using Schemes I to III. Here, 3-bromo-5-(pentafluorosulfanyl)benzaldehyde (CAS No.: 1240257-22-0), 3-amino-5-hydroxybenzoic acid, and 1,3-diamino-2-propanol are all commercially available and can be obtained.

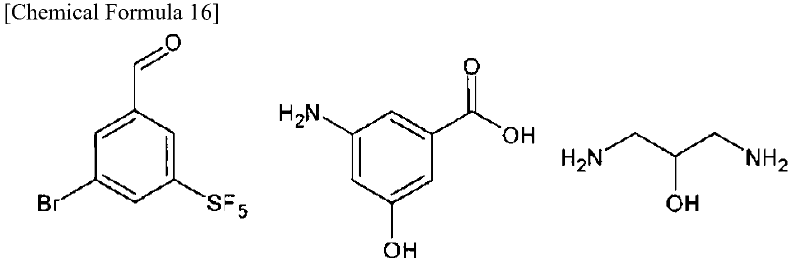

In the case of the compound IBF-13, it can be synthesized from the following compounds using Schemes I to III. Here, 3-methyl-5-(pentafluorosulfanyl)benzaldehyde (CAS No.: 1240257-01-5) and 5-aminonicotinic acid (CAS No.: 24242-19-1) are commercially available, and 2-fluoropropane-1,3-diamine can be obtained, for example, by the synthesis method described in Example 1.

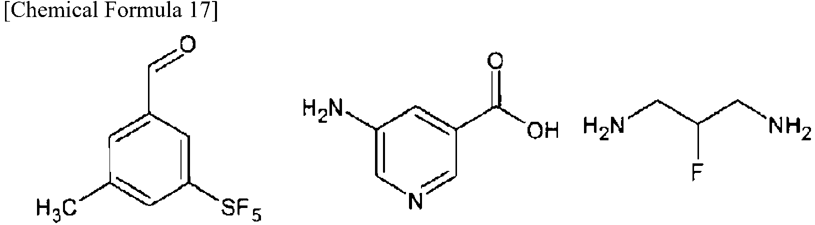

Further, as other synthesis methods, for example, synthesis methods described in JP-T-2015-524412, JP-T-2019-500355, and Neil C. Henderson et al., Nat Med., December 2013; 19(12): 1-26, and principles of organic chemistry applied by those skilled in the art can also be utilized.

### 2. Integrin Inhibitor

Integrin is known to mediate transformation of TGFβ from a latent type to an active type, and, in the present invention, the compounds serving as an active ingredient of the integrin inhibitor are compounds having an activity of inhibiting integrin function involved in fibrosis. The compounds represented by formula (I), for example, the compounds described in Table 1 or a pharmaceutically acceptable salt thereof, have an activity of inhibiting integrin function involved in fibrosis, and can be used as an integrin inhibitor. The structures of the compounds represented by formula (I) are as described in the above 1.

Integrins involved in fibrosis are, for example, αvβ1, αvβ3, αvβ5, αvβ6, αvβ8, and α8β1.

In the present invention, "inhibiting integrin function" means inhibiting binding of the cell membrane surface receptor integrin to a ligand (extracellular matrix and the like). Thereby, ligand binding of integrin and transmission signal to be transmitted to cells via integrin are blocked. By the former, cell adhesion, TGFβ activation, cell movement, cell migration, and the like are inhibited, and by the latter, cell differentiation and proliferation are inhibited.

In order to examine whether or not an activity of inhibiting integrin function is present, analyses such as, without limitation, for example, a cell adhesion inhibition test, a cell detachment test, and ligand binding inhibition of a recombinant or purified soluble integrin heterodimer, and the like are used.

### 3. Pharmaceutical Composition

The pharmaceutical composition of the present invention is a composition containing a compound represented by formula (I) or a pharmaceutically acceptable salt thereof, and a pharmaceutically acceptable carrier, and can be obtained, for example, by mixing the compound represented by formula (I) or a pharmaceutically acceptable salt thereof and a pharmaceutically acceptable carrier. The pharmaceutical composition of the present invention can treat or prevent various diseases, with inhibition of integrin function as one mechanism of action. The structure of the compound represented by formula (I) is as described in the above 1. The pharmaceutical composition of the present invention can be, for example, without limitation, a tablet, granules, a capsule, a powder, a liquid preparation, an injection, a suppository, a patch, an eye drop, or an inhalant.

"Pharmaceutically acceptable salt" can be, for example, without limitation, when an acidic functional group is present in the compound, a sodium salt, a potassium salt, an ammonium salt, and the like. Further, when a basic functional group is present in the compound, it can be a salt with hydrochloric acid, phosphoric acid, acetic acid, phthalic acid, fumaric acid, oxalic acid, and the like.

As the "pharmaceutically acceptable carrier" used in the pharmaceutical composition of the present invention, various inorganic or organic carrier substances can be used. When the pharmaceutical composition is a solid preparation such as a tablet or granules, excipients, lubricants, binders, disintegrants, and the like can be used, and when it is a liquid preparation such as a liquid agent or an injection, solvents, solubilizers, suspending agents, buffers, and the like can be used.

Further, as necessary, additives such as antioxidants, preservatives, and colorants can also be used.

Without limitation, examples of excipients include lactose, D-mannitol, starch, and the like, examples of lubricants include magnesium stearate, talc, and the like, examples of binders include crystalline cellulose, gelatin, and the like, and examples of disintegrants include carboxymethylcellulose and the like.

Further, examples of solvents include distilled water, alcohol, propylene glycol, and the like, examples of solubilizers include polyethylene glycol, ethanol, and the like, examples of suspending agents include stearyl triethanolamine, sodium lauryl sulfate, and the like, and examples of buffers include phosphates, acetates, and the like.

### 4. Therapeutic Agent/Prophylactic Agent for Disease Accompanied by Fibrosis

The therapeutic agent or prophylactic agent for a disease accompanied by fibrosis of the present invention contains, as an active ingredient, a compound represented by formula (I) or a pharmaceutically acceptable salt thereof. The structure of the compound represented by formula (I) is as described in the above 1, and the pharmaceutically acceptable salt thereof is as described in the above 3.

In the present invention, a disease accompanied by fibrosis is a disease accompanied by fibrosis of an organ or tissue, and includes pulmonary fibrosis, liver fibrosis, kidney fibrosis, pancreatic fibrosis, scleroderma, keloid, skin scarring, retinal scarring, corneal scarring, drug-induced gingival hyperplasia, frozen shoulder, arthritis, rheumatoid arthritis, and myocardial fibrosis progressing in diabetic cardiomyopathy.

In the present invention, "kidney fibrosis" includes diabetic nephropathy, chronic glomerulonephritis, and hypertensive nephrosclerosis.

In the present invention, "lung fibrosis" includes idiopathic interstitial pneumonia, collagen disease-associated pneumonia, pneumoconiosis, drug-induced interstitial pneumonia, radiation pneumonitis, and pulmonary fibrosis associated with sarcoidosis and hypersensitivity pneumonitis, and further includes acute exacerbation of any of the above interstitial pneumonias or pulmonary fibroses, and acute interstitial pneumonia.

In the present invention, "liver fibrosis" includes viral hepatitis, fatty liver, hepatitis associated with alcoholic hepatitis, hepatitis associated with non-alcoholic hepatitis (NASH), liver cirrhosis, liver fibrosis, and liver fibrosis associated with primary biliary cholangitis and autoimmune hepatitis.

The therapeutic agent or prophylactic agent for a disease accompanied by fibrosis of the present invention, by containing, as an active ingredient, a compound represented by formula (I) or a pharmaceutically acceptable salt thereof, exerts an effect of alleviating symptoms of diseases accompanied by fibrosis such as kidney fibrosis, interstitial pneumonia, or liver fibrosis, or preventing occurrence of a disease accompanied by fibrosis. Such drug efficacy is considered to be based on a mechanism of action in which the compound represented by formula (I) or a pharmaceutically acceptable salt thereof inhibits integrin function.

The compound represented by formula (I) contained as an active ingredient in the therapeutic agent or prophylactic agent for a disease accompanied by fibrosis of the present invention can have a chemical structure with a wide variety of variations in the combination of A, B, X, or Y. Therefore, in the therapeutic agent or prophylactic agent for a disease accompanied by fibrosis of the present invention, it is possible to modify the chemical structure such that drug absorption, distribution, degradability, ease of excretion, and the like are suitable.

The therapeutic agent or prophylactic agent for a disease accompanied by fibrosis of the present invention can be administered as a therapeutic agent or prophylactic agent not only to a patient diagnosed as having a disease accompanied by fibrosis, but also to a patient who may have such a disease, or a patient who may be at risk of developing such a disease.

The therapeutic agent or prophylactic agent for a disease accompanied by fibrosis of the present invention can be formulated into various dosage forms by mixing with a pharmaceutically acceptable carrier, as described in the above 3.

When used as a therapeutic agent or prophylactic agent for lung fibrosis, it can be orally administered as, for example, a tablet, granules, a capsule, a powder, a liquid preparation, and the like; however, from the viewpoint of allowing it to act directly on the lung in order to reduce side effects, it can also be an inhalant and the like. Further, depending on symptoms, the effect can be enhanced by intravenous injection.

When used as a therapeutic agent or prophylactic agent for liver fibrosis, without being limited to these dosage forms, it can be orally administered as, for example, a tablet, granules, a capsule, a powder, a liquid preparation, and the like. Further, from the viewpoint of allowing it to act directly on the liver in order to reduce side effects, it can also be directly administered to the liver by a tube or the like as an injection.

The administration route of the therapeutic agent or prophylactic agent for a disease accompanied by fibrosis of the present invention may be either oral administration or parenteral administration, and the daily dose varies depending on the type of compound, the administration method, the patient's symptoms, age, and the like. For example, in the case of oral administration, it is usually possible to administer about 0.01 to 1000 mg, more preferably about 0.1 to 500 mg, more preferably about 0.1 to 50 mg, and still more preferably about 0.1 to 10 mg, per kg body weight of a human or mammal, in 1 to several divided doses. In the case of parenteral administration such as intravenous injection, it is usually possible to administer, for example, about 0.01 mg to 300 mg, more preferably about 1 mg to 100 mg, more preferably about 1 to 50 mg, and still more preferably about 1 to 10 mg, per kg body weight of a human or mammal. For the compound of the present invention, an effect has been confirmed even at an extremely small amount of oral administration of 1 mg/kg, and it is possible to reduce the dose compared with conventional compounds. In this respect, in the case of conventional integrin inhibitory compounds, 100 mg/kg is usual, and even those of small amounts are 50 mg/kg.

The therapeutic agent or prophylactic agent for a disease accompanied by fibrosis of the present invention may contain, in addition to the compound or a pharmaceutically acceptable salt thereof according to the present invention, an active ingredient of at least one drug selected from drugs classified as therapeutic agents or prophylactic agents for diseases accompanied by fibrosis. As such active ingredients, without limitation, for example, pirfenidone, nintedanib, and the like can be used. Further, the therapeutic agent or prophylactic agent for a disease accompanied by fibrosis of the present invention can be used in combination with another therapeutic agent or prophylactic agent for a disease accompanied by fibrosis.

### [EXAMPLES]

Hereinafter, the present invention will be described in detail by Examples; however, the present invention is not limited thereto.

### (Example 1) Synthesis of IBF-17

IBF-17, which is one of the compounds of the present invention, was produced. First, (R,E)-2-methyl-N-[3-(pentafluorosulfanyl)benzylidene]propane-2-sulfinamide (Intermediate 1-1) represented by the following structural formula was synthesized by the following steps.

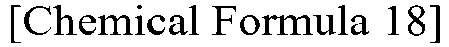

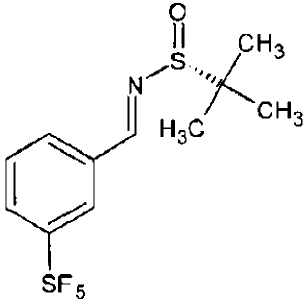

To 7.52 g of 3-(pentafluorosulfanyl)benzaldehyde were added 75 mL of anhydrous toluene, 4.12 g of (R)-2-methylpropane-2-sulfinamide, 0.130 mL of pyrrolidine, and 32 g of molecular sieves (4 Å) in a warm room, and the mixture was stirred at 70°C for 3 hours under an argon atmosphere. Further, 0.130 mL of pyrrolidine was added at room temperature, and the mixture was stirred at 70°C for 6 hours.

After completion of the reaction, the reaction solution was filtered through Celite and washed with 125 mL of toluene. The filtrate and washings were concentrated under reduced pressure, n-hexane was added, the solid was crushed, subjected to ultrasonic treatment, and then stirred for a while under an ice bath. The solid was collected by filtration, washed with cold n-hexane, and dried under reduced pressure to obtain 7.50 g of Intermediate 1-1 as a pale yellow solid. Further, the filtrate and washings were concentrated under reduced pressure, n-hexane was added, the solid was crushed, subjected to ultrasonic treatment, the solid was collected by filtration, washed with cold n-hexane, and dried under reduced pressure to obtain 0.82 g of Intermediate 1-1 as a white solid.

The results of mass spectrometry and NMR measurement spectrum of Intermediate 1-1 are as follows.
Mass spectrum (DUIS, m/z): 336 [M+H]⁺
¹H-NMR(400MHz, CDCl₃)δ: 8.62(s, 1H), 8.27-8.23(m, 1H), 8.01-7.96(m, 1H), 7.93-7.88(m, 1H), 7.61(t, J=7.9Hz, 1H), 1.29(s, 9H)

Subsequently, methyl (S)-3-[((R)-tert-butylsulfinyl)amino]-3-[3-(pentafluorosulfanyl)phenyl]propanoate (Intermediate 1-2) represented by the following structural formula was synthesized as follows.

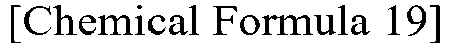

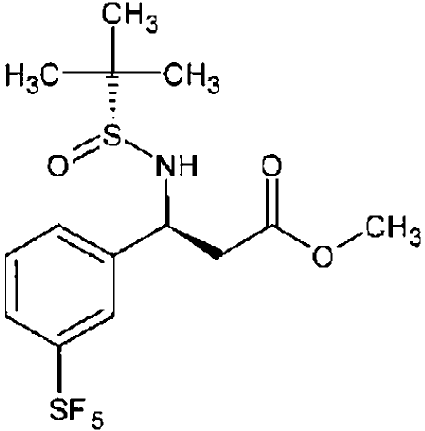

To 16.20 g of zinc was added 2.47 g of copper(I) chloride, and the mixture was degassed under reduced pressure at 70 to 80°C for 15 minutes. The atmosphere was replaced with argon, and, under an argon atmosphere, 80 mL of anhydrous tetrahydrofuran was added and the mixture was stirred at 70°C for 30 minutes. Thereafter, the mixture was cooled to 50°C, and 5.80 mL of methyl bromoacetate was added in portions so that the internal temperature did not exceed 60°C, followed by stirring at 50°C for 1 hour. After returning to room temperature, the mixture was cooled with an ice-salt bath, and a solution of 8.32 g of (R,E)-2-methyl-N-[3-(pentafluorosulfanyl)benzylidene]propane-2-sulfinamide (Intermediate 1-1) in 40 mL of anhydrous tetrahydrofuran was added dropwise under an argon atmosphere at an internal temperature of -10°C or lower, followed by rinsing with 10 mL of anhydrous tetrahydrofuran, and stirring for 2.5 hours under an ice bath.

After completion of the reaction, the reaction solution was filtered through Celite and washed with 130 mL of tert-butyl methyl ether. To the filtrate and washings was added 110 mL of a 10% aqueous citric acid solution, and, after stirring the mixture at room temperature for a while, it was separated. The organic layer was sequentially washed with 70 mL of saturated brine, 70 mL of saturated aqueous sodium bicarbonate, and 70 mL of saturated brine, dried over anhydrous sodium sulfate, and filtered, and the filtrate was concentrated under reduced pressure. The concentrated residue thus obtained was purified by column chromatography (silica gel; elution solvent: 1,2-dichloroethane:ethyl acetate) to obtain 8.89 g of Intermediate 1-2 as a white solid.

The results of mass spectrometry and NMR measurement spectrum of Intermediate 1-2 are as follows.
Mass spectrum (DUIS, m/z): 410 [M+H]⁺
¹H-NMR (400MHz, DMSO-d₆) δ:7.92-7.88(m, 1H), 7.82-7.77(m, 1H), 7.69-7.64(m, 1H), 7.61-7.54 (m, 1H), 5.80(d, J=6.1Hz, 1H), 4.82-4.75(m, 1H). 3.55(s, 3H), 3.04 (dd, J=7.2, 15.7Hz, 1H), 2.84(dd, J=7.3, 15.7Hz, 1H), 1.07(s, 9H)_{∘}

Further, methyl (S)-3-amino-3-[3-(pentafluorosulfanyl)phenyl]propanoate hydrochloride (Intermediate 1-3) represented by the following structural formula was synthesized as follows.

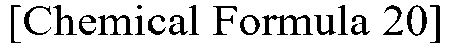

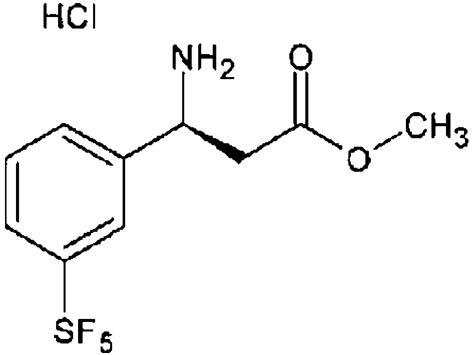

To 8.66 g of methyl (S)-3-[((R)-tert-butylsulfinyl)amino]-3-[3-(pentafluorosulfanyl)phenyl]propanoate (Intermediate 1-2) were added 90 mL of anhydrous diethyl ether, 0.86 mL of anhydrous methanol, and 47.0 mL of a 1M hydrogen chloride diethyl ether solution at room temperature under an argon atmosphere, and the mixture was stirred at room temperature for 1 hour.

After completion of the reaction, the reaction solution was ice-cooled, and the precipitated solid was collected by filtration, washed with anhydrous diethyl ether, and dried under reduced pressure to obtain 7.10 g of Intermediate 1-3 as a white solid.

The results of mass spectrometry and NMR measurement spectrum of Intermediate 1-3 are as follows.
Mass spectrum (DUIS, m/z): 306 [M+H]⁺
¹H-NMR (400MHz, DMSO-d₆) δ:8.82(br s, 3H), 8.18-8.14(m, 1H), 7.96-7.87(m, 2H), 7.72-7.65(m, 1H), 4.79 (dd, J=6.0, 8.5Hz, 1H), 3.56(s, 3H), 3.24(dd, J=6.0, 16.5Hz, 1H), 3.08 (dd, J=8.5, 16.5Hz, 1H)_{∘}

Further, 2-fluoromalonamide (Intermediate 1-9) represented by the following structural formula was synthesized as follows.

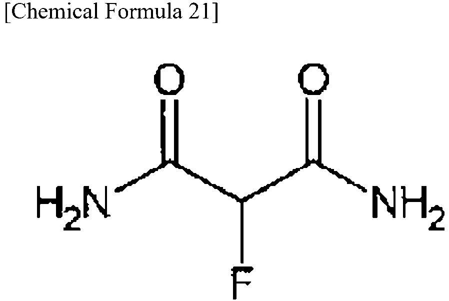

To a methanol (100 mL) solution of 25.0 g of diethyl 2-fluoromalonate was added 80 mL of a 7 N ammonia methanol solution at room temperature under an argon stream, and the mixture was stirred at room temperature for 4 hours.

After completion of the reaction, the solid was collected by filtration, washed with methanol, and dried under reduced pressure at 50°C to obtain 15.91 g of Intermediate 1-9 as a white solid.

The result of NMR measurement spectrum of Intermediate 1-9 is as follows. ¹H-NMR (400MHz, DMSO-d₆) δ:7.78-7.48(m, 4H), 5.16(d, J=48.9Hz, 1H)_{∘}

Then, 2-fluoropropane-1,3-diamine hydrochloride (Intermediate 1-10) represented by the following structural formula was synthesized as follows.

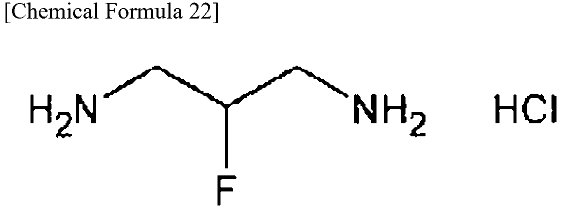

To 400 mL of a 0.9 M borane-tetrahydrofuran solution was added 7.20 g of 2-fluoromalonamide (Intermediate 1-9) in portions at room temperature under an argon stream, and the mixture was stirred at room temperature for 20 hours.

After completion of the reaction, the reaction solution was cooled to 0°C, and 73 mL of methanol was added dropwise at 20°C or lower. The mixture was stirred at room temperature for 1 hour and concentrated under reduced pressure. To the concentrated residue was added 40 mL of ethanol, and 60 mL of a 2 M hydrogen chloride ethanol solution was added at 0°C, followed by stirring at room temperature for 3 hours. The precipitated solid was collected by filtration, the solid was dissolved in methanol, and concentrated under reduced pressure. To a methanol (200 mL) solution of 6.91 g of the obtained solid was added 200 mL of water, and 70 g of Amberlite (IRA478RF CL) washed sequentially with 200 mL of water and 200 mL of methanol was added thereto at room temperature, followed by stirring at room temperature for 30 minutes. The reaction solution was filtered, and the filtrate was concentrated under reduced pressure to obtain 4.55 g of Intermediate 1-10 as a white solid.

The result of NMR measurement spectrum of Intermediate 1-10 is as follows. ¹H-NMR(400MHz, DMSO-d₆+D₂O) δ: 4.77-4.53(m, 1H), 3.01-2.79 (m, 4H)_{∘}

Further, 3-[(5-fluoro-1,4,5,6-tetrahydropyrimidin-2-yl)amino]-5-hydroxybenzoic acid (Intermediate 1-6) represented by the following structural formula was synthesized as follows.

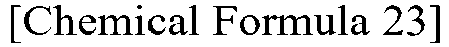

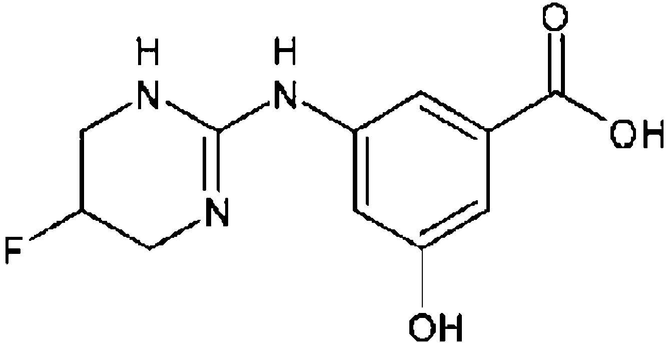

To an anhydrous N,N-dimethylformamide (6 mL) solution of 1.0 g of 3-amino-5-hydroxybenzoic acid was added, under an argon atmosphere, an anhydrous N,N-dimethylformamide (6 mL) solution of 0.60 g of methyl isothiocyanate at room temperature, and the mixture was stirred at room temperature for 30 minutes, and then stirred at 60°C for 17.5 hours. Thereafter, the reaction solution was cooled to room temperature, 1.0 mL of iodomethane was added under an ice bath, and the mixture was stirred at room temperature for 1.5 hours, and the reaction solution was concentrated under reduced pressure. To the obtained concentrated residue were added 2.09 g of 2-fluoropropane-1,3-diamine hydrochloride (Intermediate 1-10) and 10 mL of anhydrous N,N-dimethylformamide under an ice bath, and the mixture was stirred at 50°C for 30 minutes and then stirred at 95°C for 4.5 hours.

After completion of the reaction, the reaction solution was cooled to room temperature and added to 10 mL of water. Under an ice bath, 6 N hydrochloric acid was added to adjust the pH to 6.0. The solvent was partially distilled off by concentration under reduced pressure, and the mixture was stirred overnight at room temperature. The precipitated solid was collected by filtration, washed sequentially with water and acetonitrile, and dried under reduced pressure to obtain 356 mg of Intermediate 1-6 as a white solid.

The results of mass spectrometry and NMR measurement spectrum of Intermediate 1-6 are as follows.
Mass spectrum (ESI, m/z): 254 [M+H]⁺
¹H-NMR(400MHz, DMSO-d₆+D₂O) δ: 7.19-7.13(m, 2H), 6.76-6.70(m, 1H), 5.43-5.17(m, 1H), 3.73-3.40(m, 4H)_{∘}

Subsequently, {3-[(5-fluoro-1,4,5,6-tetrahydropyrimidin-2-yl)amino]-5-hydroxybenzoyl}glycine (Intermediate 1-7) represented by the following structural formula was synthesized as follows.

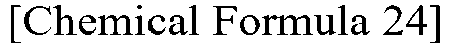

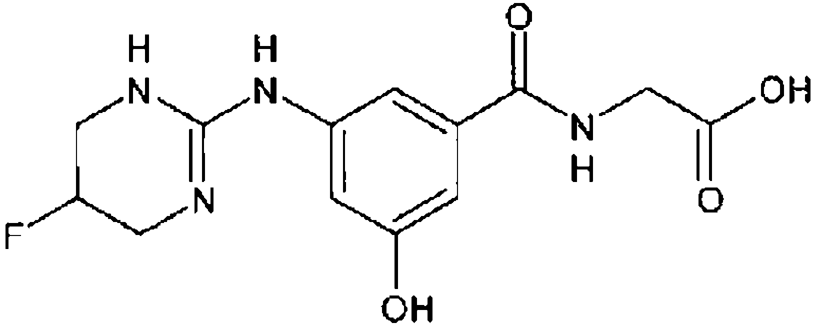

To a N,N-dimethylformamide (1.5 mL)-dichloromethane (1.5 mL) suspension of 300 mg of 3-[(5-fluoro-1,4,5,6-tetrahydropyrimidin-2-yl)amino]-5-hydroxybenzoic acid (Intermediate 1-6) were added, under an argon stream, 182 mg of ethyl glycinate hydrochloride and 0.22 mL of N,N'-diisopropylcarbodiimide at room temperature, and the mixture was stirred at room temperature for 19 hours. Thereafter, 3.6 mL of a 2N aqueous sodium hydroxide solution was added and the mixture was stirred at room temperature for 1.5 hours.

After completion of the reaction, dichloromethane was added to the reaction solution and the mixture was separated. To the aqueous layer was added 6 N hydrochloric acid to adjust the pH to 5.0, followed by stirring at room temperature for 2 hours. The precipitated solid was collected by filtration, washed sequentially with water and acetonitrile, and dried under reduced pressure at 60°C to obtain 188 mg of Intermediate 1-7 as a white solid.

Thereafter, methyl (3S)-3-(2-{3-[(5-fluoro-1,4,5,6-tetrahydropyrimidin-2-yl)amino]-5-hydroxybenzamido}acetamido)-3-[3-(pentafluorosulfanyl)phenyl]propanoate trifluoroacetate (Intermediate 1-8) represented by the following structural formula was synthesized as follows.

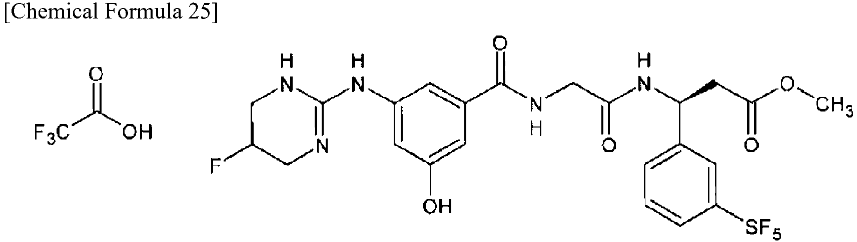

To a N,N-dimethylformamide (3 mL) suspension of 300 mg of {3-[(5-fluoro-1,4,5,6-tetrahydropyrimidin-2-yl)amino]-5-hydroxybenzoyl}glycine (Intermediate 1-7) were added, under an argon stream, 363 mg of methyl (S)-3-amino-3-[3-(pentafluorosulfanyl)phenyl]propanoate hydrochloride (Intermediate 1-3) and 144 mg of 1-hydroxybenzotriazole at room temperature, and the mixture was stirred at room temperature for 10 minutes. Then, 0.18 mL of N,N'-diisopropylcarbodiimide was added at room temperature, and the mixture was stirred at room temperature for 15 hours.

After completion of the reaction, the reaction solution was purified by silica gel chromatography (silica gel; elution solvent: 0.1% TFA acetonitrile:0.1% TFA aqueous solution) to obtain 673 mg of Intermediate 1-8 as a pale yellow solid.

The results of mass spectrometry and NMR measurement spectrum of Intermediate 1-8 are as follows.
Mass spectrum (ESI, m/z): 598 [M+H]⁺
¹H-NMR (400MHz, DMSO-d₆) δ:10.05(br s,1H), 9.78(s, 1H), 8.69-8.63(m, 2H), 8.28(br s, 2H), 7.87-7.84(m, 1H), 7.82-7.76(m, 1H), 7.68-7.63(m, 1H), 7.62-7.56(m, 1H), 7.17-7.14(m, 1H), 7.14-7.11(m, 1H), 6.75(t, J=2.0Hz, 1H), 5.38-5.18(m, 2H), 3.94-3.79(m, 2H), 3.60-3.39(m, 7H), 2.86(d, J=7.4Hz, 2H)_{∘}

Finally, (3S)-3-(2-{3-[(5-fluoro-1,4,5,6-tetrahydropyrimidin-2-yl)amino]-5-hydroxybenzamido}acetamido)-3-[3-(pentafluorosulfanyl)phenyl]propanoic acid (IBF-17) represented by the following structural formula was synthesized as follows.

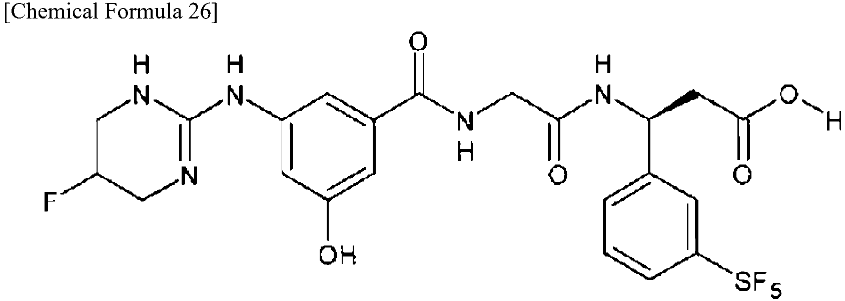

To an acetonitrile (3.5 mL)/water (3.5 mL) solution of 670 mg of methyl (3S)-3-(2-{3-[(5-fluoro-1,4,5,6-tetrahydropyrimidin-2-yl)amino]-5-hydroxybenzamido}acetamido)-3-[3-(pentafluorosulfanyl)phenyl]propanoate trifluoroacetate (Intermediate 1-8) was added 113 mg of lithium hydroxide at room temperature under an argon stream, and the mixture was stirred at room temperature for 2 hours.

After completion of the reaction, 1N hydrochloric acid was added to the reaction solution to adjust the pH to 5.8. After filtration, the filtrate was concentrated under reduced pressure, acetonitrile was distilled off under reduced pressure, and the mixture was stirred at room temperature for 17 hours. A small amount of acetonitrile was added to obtain a homogeneous solution, and purification was performed using BondElut (elution solvent: water:acetonitrile:methanol = 100:0:0 to 0:100:0 to 0:67:33) to obtain 426 mg of IBF-17 as a light brown solid.

The results of mass spectrometry and NMR measurement spectrum of IBF-17 are as follows.
Mass spectrum (ESI, m/z): 584 [M+H]⁺
¹H-NMR (400MHz, CD₃OD) δ:7.83 (t, J=1.8Hz, 1H), 7.68-7.58(m, 2H), 7.51-7.42(m, 1H), 7.23-7.20(m, 1H), 7.20-7.17(m, 1H), 6.78(t, J=2.1Hz, 1H), 5.28(t, J=5.5Hz, 1H), 5.25-5.08(m, 1H), 4.09(d, J=16.9Hz, 1H), 4.01(d, J=16.9Hz, 1H) ,3.70-3.51(m, 3H), 3.50-3.40(m, 1H), 2.70(dd, J=5.5, 15.3Hz, 1H), 2.63(dd, J=5. 5, 15.3Hz, 1H)_{∘}

### (Example 2) Effect on TGFβ Activation

In this Example, lung fibroblasts were used as integrin-expressing cells, and the effect of IBF-17, which is a compound of the present invention, on TGFβ activity was examined. Fibroblasts isolated from lungs of male Wistar rats and cultured for 7 days were seeded at 2×10³ cells per well and incubated for 5 days. After 5 days, IBF-17 or C8 (a known αvβ1 inhibitor) was added so as to give final concentrations of 2, 20, or 200 nM, and 2 hours thereafter, TGFβ-responsive luciferase-producing reporter cells CCL64 were seeded at 2×10^4 cells and cocultured for 18 hours. After that, the cells were lysed, luciferase assay reagent (Promega Corporation) was added, and luciferase activity was measured using a luminometer. The results are shown in FIG. 1. The structure of C8, which is a known αvβ1 inhibitor, is as follows.

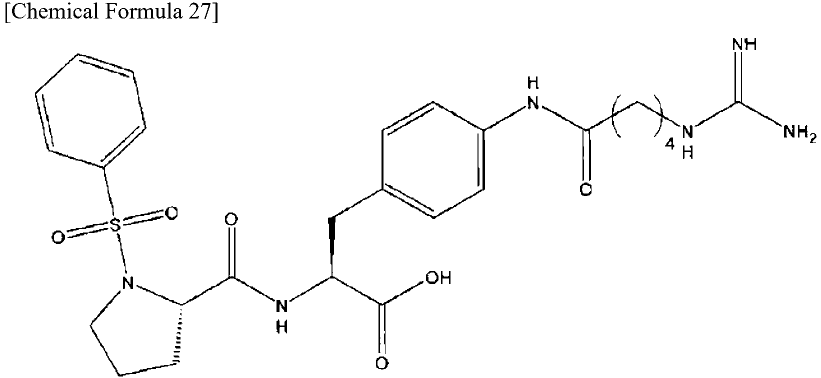

FIG. 1 shows, as a comparison, the results for reporter cells alone at the left end, to the right thereof a mixed culture of reporter cells and lung fibroblasts, and further to the right the results when each of IBF-17 and C8 was added at 2, 20, and 200 nM. In the present experimental results, IBF-17 suppressed activation of TGFβ in a concentration-dependent manner in the concentration range of 2 to 200 nM, and at 200 nM the luciferase value was close to the value for reporter cells alone, and TGFβ activation by integrin expressed in lung fibroblasts could be almost completely inhibited. On the other hand, in the case of C8, which is an αvβ1-selective inhibitor, inhibition was not observed at 2 nM, and although inhibition of TGFβ activation was observed at 20 nM and 200 nM, the difference in inhibitory effect was clear as compared with IBF-17 (IBF-17 had a markedly higher inhibitory effect).

### (Example 3) Effect on Differentiation of Fibroblasts into Myofibroblasts

In this Example, the effect of IBF-17, which is a compound of the present invention, on differentiation of fibroblasts into myofibroblasts was examined. The medium was replaced with a serum-free medium, and rat lung-derived fibroblasts isolated in Example 2 were cultured for 24 hours. After culturing, IBF-17 was added so as to give final concentrations of 2, 20 nM, or 200 nM, and C8 was added so as to give final concentrations of 2, 20, or 200 nM, followed by reaction for 2 hours. After the reaction, recombinant human TGFβ1 was added so as to give a final concentration of 5 ng/mL, and stimulation was carried out for 96 hours to induce differentiation into myofibroblasts. After induction of differentiation, protein was extracted from the cells and western blotting was performed, a band of α-SMA, which is a differentiation marker, was detected, and the band was quantified as a relative band intensity with respect to glyceraldehyde-3-phosphate dehydrogenase (GAPDH), which is a control.

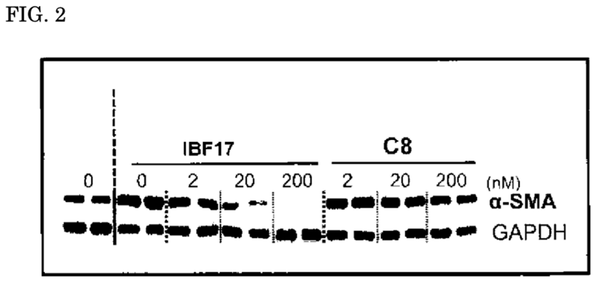

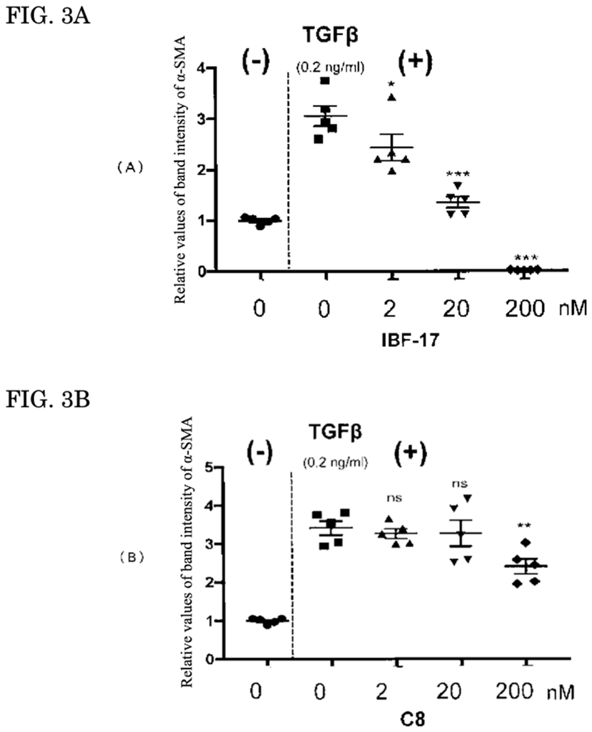

FIG. 2 shows results of the western blotting using an anti-α-smooth muscle actin antibody (α-SMA) and an anti-GAPDH antibody. FIG. 3 shows numerical values of the band intensity, and is represented as relative values with respect to the band intensity of cells without addition of TGFβ.

From this Example, differentiation of fibroblasts into myofibroblasts (by 0.2 ng/mL TGFβ) was evaluated using α-smooth muscle actin as marker. In the case of addition of IBF-17 in FIG. 3(A), inhibition of differentiation was observed at a concentration of 2 nM, and at a concentration of 20 nM, inhibition was 80% or more. On the other hand, in FIG. 3(B), the effect of addition of C8 was similarly observed, but there was no effect at concentrations of 2 and 20 nM, and suppression was finally observed at 200 nM.

C8 used as a control for IBF-17 in Example 2 and Example 3 was inferior to the effect of IBF-17 in TGFβ activation and myofibroblast differentiation, which are markers for estimating fibrotic action in vitro; however, since C8 alone suppresses in vivo fibrosis of liver, lung, kidney, and heart (The αvβ1 integrin plays a critical in vivo role in tissue fibrosis, Nilgun I Reed, Hyunil Jo, Chun Chen, Kazuyuki Tsujino, Thomas D Arnold, William F DeGrado, Dean Sheppard. Sci Transl Med 20; 8(288): 288ra79, 2015), an in vivo effect greater than that is expected for IBF-17.

### (Example 4-1) 8-Week Administration Experiment in Carbon Tetrachloride (CCl4) Administration Model

In this Example, over a period of 8 weeks, carbon tetrachloride (CCl4) for inducing fibrosis in the liver and compounds of the present invention (IBF-17, IBF-18, and IBF-20) were administered to mice, and the state of tissue fibrosis was observed.

To mice, carbon tetrachloride (diluted 4-fold with olive oil) was intraperitoneally administered at 1 mL/kg twice per week (for example, Monday and Friday, or Tuesday and Friday) for a total of 8 weeks. Throughout the entire test period, IBF-17, IBF-18, or IBF-20 was orally administered daily at 1 or 10 mg/kg twice per day (with an interval of 8 hours). After completion of the experiment, the liver was collected, and the amount of collagen was quantified by hydroxyproline measurement, and a specimen was stained with Sirius red to quantify the fibrotic area. In addition, expression of fibrosis markers, Col1a1 and Acta2 (α-SMA) genes, was analyzed by qPCR.

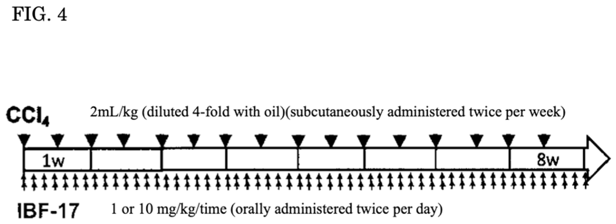

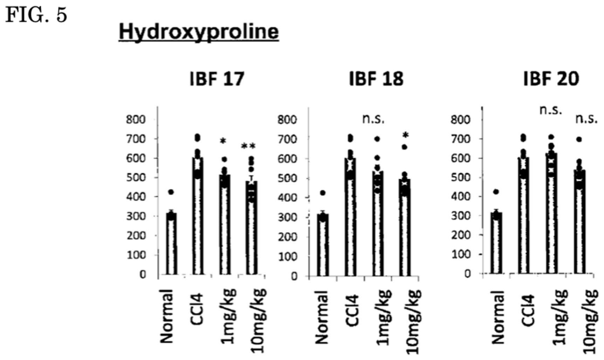

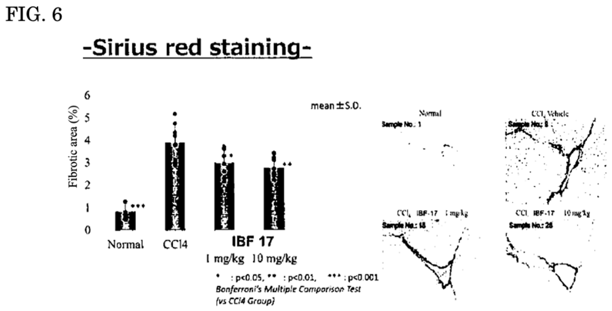

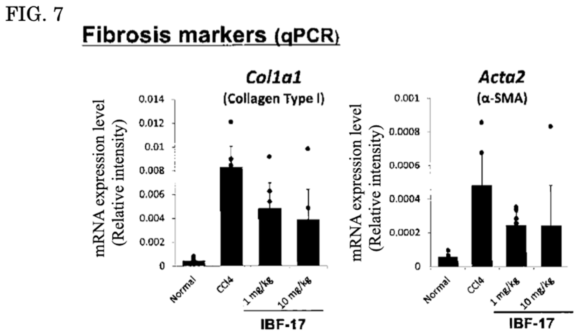

FIG. 4 shows the dosing schedule, FIG. 5 shows hydroxyproline quantification results of liver tissue, FIG. 6 shows comparison of Sirius red-stained area of tissue and photographs after staining, and FIG. 7 shows mRNA expression levels (relative intensity) of fibrosis marker genes (Col1a1 gene and Acta2 gene). From FIG. 5, it can be confirmed that administration of IBF-17 and IBF-18 decreased the amount of hydroxyproline in mice, but that, in administration at 10 mg/kg, such an effect was not observed for IBF-20. When comparing IBF-17 and IBF-18, for which an effect was observed, only IBF-17 showed an effect at 1 mg/kg. Therefore, for the IBF-17 administration group, decreases in Sirius red-stained area and fibrosis markers in liver tissue were examined. In both the 1 mg/kg and 10 mg/kg administration groups of IBF-17, the Sirius red-stained area was decreased as compared with the non-administration group, and the degree of decrease followed the dose. The right side of FIG. 6 shows staining examples, and in all of them sufficient staining is observed. From FIG. 7, it can be read that expression of both Col1a1 and Acta2 tends to be decreased by IBF-17.

### (Example 4-2) 3-Day Administration Experiment in Carbon Tetrachloride (CCl4) Administration Model

In this Example, carbon tetrachloride (CCl4) for inducing fibrosis in the liver was administered once to mice, and thereafter, the compound of the present invention (IBF-17) was administered for 3 days, and then the liver was collected and observed. More specifically, carbon tetrachloride (diluted 4-fold with olive oil) was intraperitoneally administered once at 2 mL/kg to mice that had been fasted for half a day from the day before the test. IBF-17 was subcutaneously administered daily at 1 mg/kg twice per day (with an interval of 8 hours), including the day of carbon tetrachloride administration. At 72 hours after carbon tetrachloride administration, the mice were dissected and the liver was collected, and expression of fibrosis marker genes (Acta2, Col1a1, Col3a1, Tgfb1, Loxl1, and Ddr2) was analyzed by qPCR.

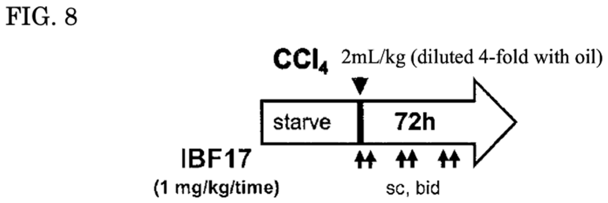

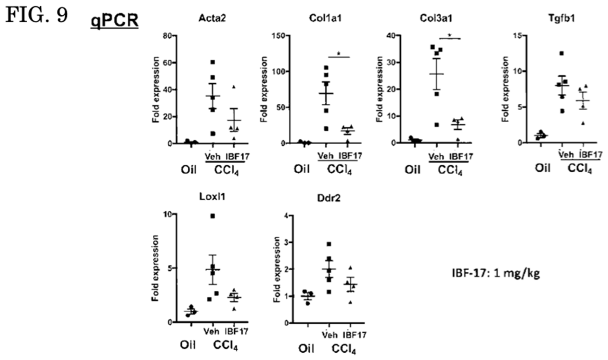

FIG. 8 shows the dosing schedule, and FIG. 9 shows mRNA expression levels (relative intensity) measured by qPCR. FIG. 9 shows analysis results for six fibrosis markers, and it is understood that each marker increased 72 hours after CCl4 administration. However, it is observed that all of these markers were decreased by administration of IBF-17, and among them, the two collagen species Col1a1 and Col3a1 were clearly decreased.

### (Example 5) 8-Week Administration Experiment in NASH Model

In this Example, over a period of 8 weeks, while providing mice with CDAHFD (choline-deficient amino acid-restricted high-fat diet), compounds of the present invention (IBF-17, IBF-18, and IBF-20) were administered, and the state of tissue fibrosis was observed. More specifically, mice were fed with a special diet CDAHFD (Research Diets) for inducing a human NASH-like pathological condition for a total of 8 weeks, replacing with new diet twice per week (for example, Monday and Friday, or Tuesday and Friday). Throughout the entire test period, IBF-17, IBF-18, or IBF-20 was orally administered daily at 1, 3, or 10 mg/kg twice per day (with an interval of 8 hours). After completion of the experiment, the amount of collagen in the liver was quantified by hydroxyproline measurement, and, for IBF-17, in addition to staining specimens with Sirius red, which specifically stains collagen, to quantify the fibrotic area, expression of genes regarded as fibrosis markers (Acta2, Col1a1, and Timp-1) was analyzed by qPCR. In addition, body weight of each mouse during the feeding period (CDAHFD temporarily decreases at the initial stage of feeding) was measured, and the weight of the collected liver was measured.

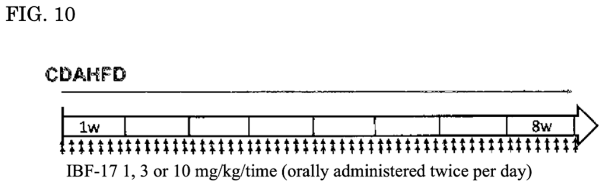

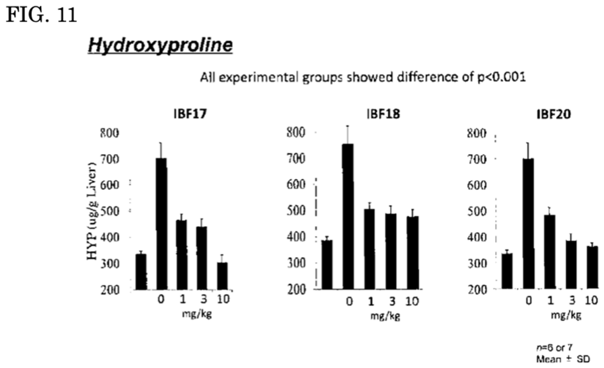

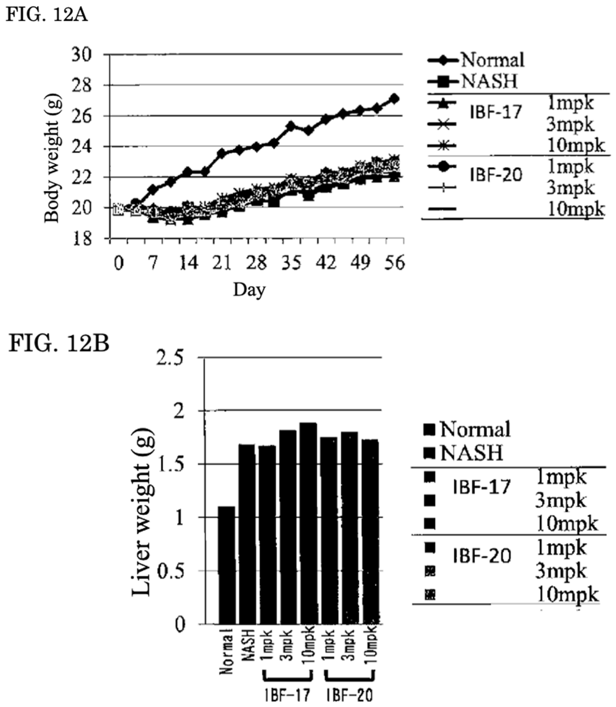

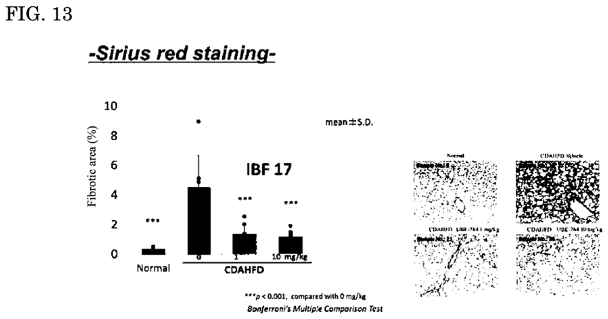

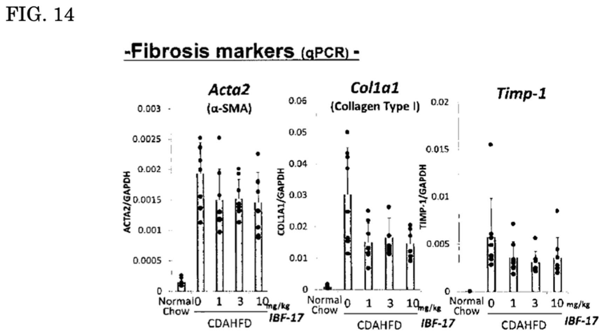

FIG. 10 shows the dosing schedule, FIG. 11 shows hydroxyproline quantification results of liver tissue (p<0.001), FIGS. 12(A) and 12(B) show changes in body weight of mice and liver weight after 56 weeks, FIG. 13 shows comparison of Sirius red-stained area of tissue and photographs after staining, and FIG. 14 shows mRNA expression levels (relative intensity) of genes regarded as fibrosis markers (Acta2 gene, Col1a1 gene, and Timp-1 gene). From FIG. 11, for all compounds (IBF-17, IBF-18, and IBF-20), decreases in hydroxyproline amount were observed at any dose (1, 3, and 10 mg/kg) as compared with the case of no administration. CDAHFD, which is a diet used for producing NASH model mice, causes fat deposition in the liver, and after administration the liver swells and increases in weight. FIG. 12 shows liver weight at the end of the experiment. By CDAHFD administration, liver weight increased to 1.5 times or more of that of the normal diet group, and the liver weight similarly increased at any dose (1, 3, and 10 mpk (mg/kg)) of any drug, and there is no effect of the drugs on liver weight. From FIG. 13 and FIG. 14, IBF-17 reduced the fibrotic area in tissue specimens as compared with the non-administration group and uniformly decreased fibrosis markers at any of 1, 3, and 10 mg/kg administration. Notably, even at only 1 mg/kg administration, an effect similar to that of 3 and 10 mg/kg administration was exerted.

### (Example 6) 10-Day Administration Experiment in Unilateral Ureteral Obstruction (UUO) Model

In this Example, over a period of 10 days, the compound of the present invention (IBF-17) was administered to mice in which the ureter on one side was ligated, and the state of tissue fibrosis was observed. More specifically, mice under deep anesthesia were laparotomized, and two sites of the left ureter were ligated using a nylon thread, and the abdomen was sutured. From 1 day after ligation, IBF-17 was subcutaneously administered daily at 1 or 5 mg/kg twice per day (with an interval of 8 hours) for 9 days. At 10 days after ligation, the kidney was collected and expression of genes regarded as fibrosis markers (Acta2, Col1a1, Col3a1, Ddr2, Tgfb1, Tnc, Trpc6, and Loxl1) was analyzed by qPCR.

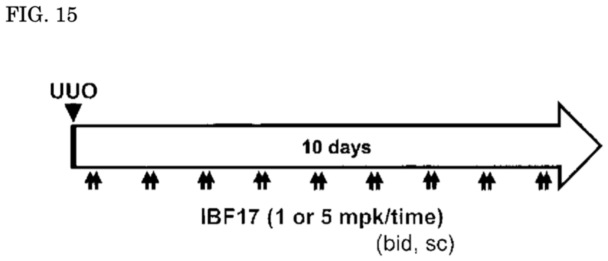

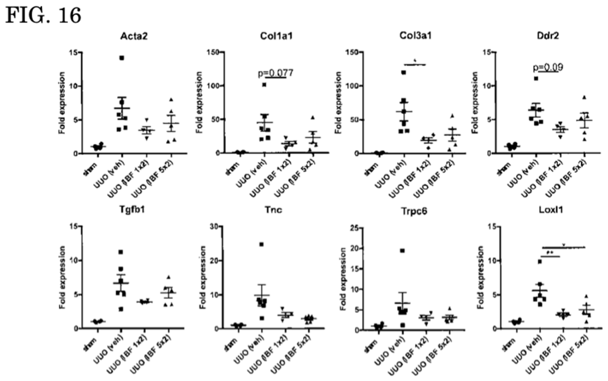

FIG. 15 shows the dosing schedule, and FIG. 16 shows mRNA expression levels (relative intensity) by qPCR. Among eight fibrosis markers, Acta2, Tgfb1, Tnc, and Trpc6 each showed a decreasing tendency, and Col1a1, Col3a1, Ddr2, and Loxl1 showed significant decreases. From this Example, it was confirmed that IBF-17 has an effect also on kidney fibrosis.

### (Example 7) Examination of Effective Dose and Nontoxic Dose

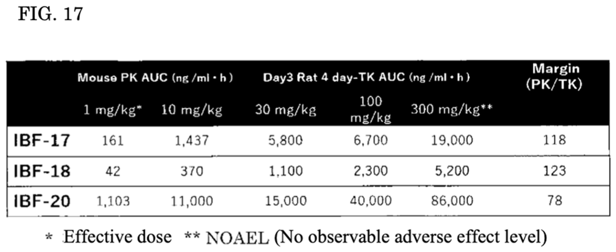

FIG. 17 shows the area under the blood concentration-time curve (Area under the curve: AUC) when the compounds of the present invention (IBF-17, IBF-18, and IBF-20) were administered at 1, 10, 30, 100, or 300 mg/kg, and a margin comparing the effective dose (1 mg/kg) and the no observable adverse effect level (NOAEL). From these results, it is expected that the compounds of the present invention have a very large margin (PK/TK) between the effective dose and the no observable adverse effect level (toxic dose), and the risk of causing side effects is extremely low.

### (Example 8) 15-Day Administration Experiment in Carbon Tetrachloride (CCl4) Administration Model

In this Example, over a period of 6 weeks, carbon tetrachloride (CCl4) for inducing fibrosis in the liver was administered to mice, and the compound of the present invention (IBF-17) was administered for a total of 15 days over 3 weeks in the latter half of the test, and the state of tissue fibrosis was observed. While Example 4 is examination of a prophylactic effect in which the compound is administered from the start of the test, this Example is examination of a therapeutic effect in which the compound is administered after 3 weeks have passed since administration of carbon tetrachloride (in a state where liver fibrosis has occurred). Specifically, to mice, carbon tetrachloride (diluted 4-fold with olive oil) was intraperitoneally administered at 1 mL/kg twice per week (for example, Monday and Friday, or Tuesday and Friday) for a total of 6 weeks. From week 4 of the start of the test, IBF-17 was orally administered at 5 mg/kg twice per day (with an interval of 8 hours) for 5 days per week (Monday to Friday). After completion of the experiment, the liver was collected and the amount of collagen was quantified by hydroxyproline measurement.

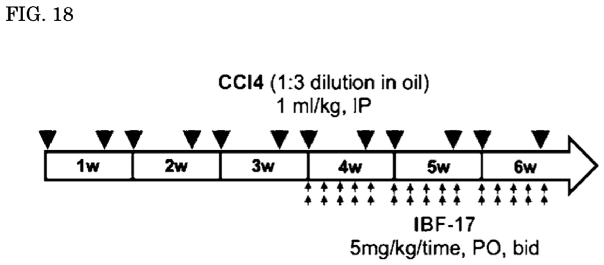

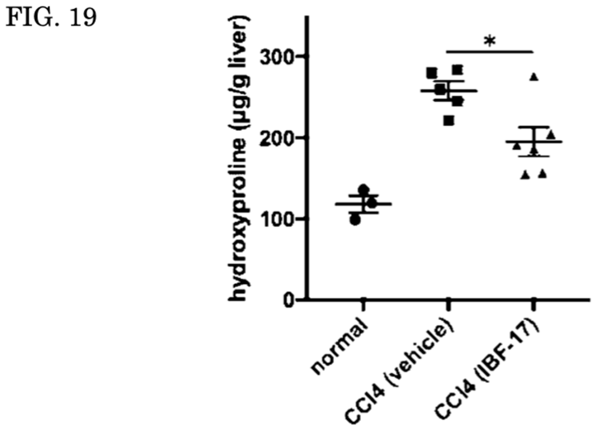

FIG. 18 shows the dosing schedule, and FIG. 19 shows hydroxyproline quantification results of liver tissue. IBF-17 significantly decreased the amount of hydroxyproline in mouse liver, and it was confirmed in this test that IBF-17 has a therapeutic effect on liver fibrosis.

### (Example 9) 10-Day Administration Experiment in Bleomycin Administration Model

In this Example, to mice administered with bleomycin for inducing fibrosis in the lung, the compound of the present invention (IBF-17) was administered for a total of 10 days over a period of 2 weeks from 1 week after bleomycin administration, and the state of tissue fibrosis was observed.

Specifically, under deep anesthesia, the neck of mice was incised, bleomycin (diluted with physiological saline) was intratracheally administered once at 1.5 U/kg, and the neck was sutured. From 1 week after bleomycin administration (the time when fibrosis begins to occur), IBF-17 was orally administered at 5 mg/kg twice per day (with an interval of 8 hours) for 5 days per week (Monday to Friday). After completion of the experiment, the lung was collected and expression of fibrosis-related genes (Acta2, Col1a1, Col3a1, and Tnc) was analyzed by qPCR. Further, lung sections were prepared, collagen was stained by Masson-trichrome staining, and the fibrotic area was quantified.

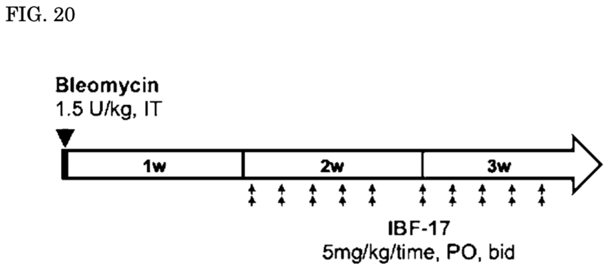

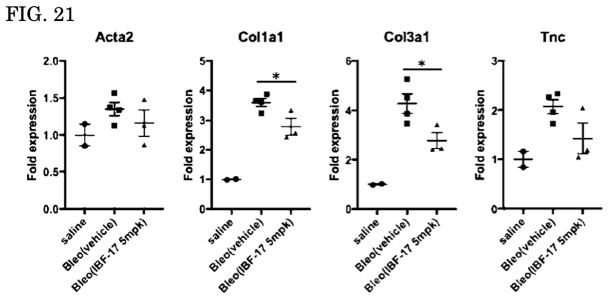

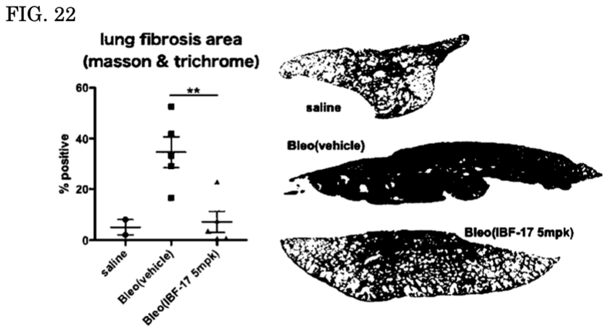

FIG. 20 shows the dosing schedule, FIG. 21 shows the results of qPCR, and FIG. 22 shows the quantified results of fibrotic area and photographs of the lung after staining (entire connected photograph of the left lobe). Among the fibrosis-related genes, expression of Col1a1 and Col3a1 was significantly decreased by administration of IBF-17. Further, the fibrotic area was also significantly decreased by administration of IBF-17. From these results, it was confirmed that IBF-17 has a therapeutic effect also on lung fibrosis.

The above detailed description merely illustrates the purpose and subject matter of the present invention, and does not limit the appended claims. Various modifications and substitutions to the described embodiments will be apparent to those skilled in the art from the teachings described herein without departing from the appended claims.

This application claims priority based on Japanese Patent Application No. 2023-102623 (filing date: June 22, 2023) filed in Japan, and the contents thereof are incorporated herein by reference. The entire contents of Japanese Patent Application No. 2023-102623 are incorporated herein and constitute a part of this specification.

### [INDUSTRIAL APPLICABILITY]

By the present invention, a novel compound that inhibits integrin function has been provided.

The present invention is useful for treatment or prevention of a disease accompanied by fibrosis.

## Claims

1. A compound represented by formula (I) or a salt thereof: 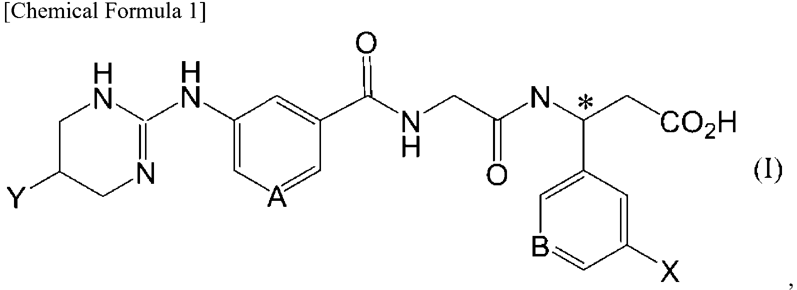
wherein A is C-OH or N,
B is C-H, C-COOH, C-CH₃, C-Br, or N,
X is -SF₅ or -Si(CH₃)₃,
Y is -F or -OH, and
* indicates an asymmetric carbon atom.

2. The compound or a salt thereof according to claim 1, wherein X is -SF₅ and B is C-H, C-CH₃, or C-Br.

3. The compound or a salt thereof according to claim 2, wherein Y is -F.

4. The compound or a salt thereof according to claim 3, wherein A is C-OH.

5. The compound or a salt thereof according to claim 1, wherein X is -Si(CH₃) ₃ and B is C-COOH or N.

6. An integrin inhibitor comprising the compound or a pharmaceutically acceptable salt thereof according to any one of claims 1 to 5.

7. A pharmaceutical composition comprising the compound or a pharmaceutically acceptable salt thereof according to any one of claims 1 to 5, and a pharmaceutically acceptable carrier.

8. A therapeutic agent or prophylactic agent for a disease accompanied by fibrosis, containing, as an active ingredient, the compound or a pharmaceutically acceptable salt thereof according to any one of claims 1 to 5.

9. The therapeutic agent or prophylactic agent for a disease accompanied by fibrosis according to claim 8, wherein the disease accompanied by fibrosis is kidney fibrosis, lung fibrosis, or liver fibrosis.

10. The therapeutic agent or prophylactic agent for a disease accompanied by fibrosis according to claim 8, for treating or preventing a disease accompanied by fibrosis in combination with at least one drug selected from drugs classified as therapeutic agents or prophylactic agents for diseases accompanied by fibrosis.

11. A therapeutic agent or prophylactic agent for a disease accompanied by fibrosis comprising a combination of the compound or a pharmaceutically acceptable salt thereof according to any one of claims 1 to 5 and an active ingredient of at least one drug selected from drugs classified as therapeutic agents or prophylactic agents for diseases accompanied by fibrosis.

12. The compound or a pharmaceutically acceptable salt thereof according to any one of claims 1 to 5, for use as a therapeutic drug or prophylactic drug for a disease accompanied by fibrosis.

13. Use of the compound or a pharmaceutically acceptable salt thereof according to any one of claims 1 to 5, for the manufacture of a medicament for treating or preventing a disease accompanied by fibrosis.

14. A method for treating or preventing a disease accompanied by fibrosis by administering the compound or a pharmaceutically acceptable salt thereof according to any one of claims 1 to 5 to a patient.
